(19) Europäisches Patentamt
European Patent Office
Office européen des brevets

(11) EP 0 739 887 A1

# (12) DEMANDE DE BREVET EUROPEEN

(43) Date de publication:
**30.10.1996 Bulletin 1996/44**

(21) Numéro de dépôt: **96400872.6**

(22) Date de dépôt: **24.04.1996**

(51) Int Cl.[6]: **C07D 303/16**, A61K 31/335, C07D 411/08, C07C 271/64 // (C07D411/08, 303:16, 317:64)

(84) Etats contractants désignés:
**AT BE CH DE DK ES FI FR GB GR IE IT LI LU NL PT SE**

(30) Priorité: **27.04.1995 FR 9505052**

(71) Demandeur: **ADIR ET COMPAGNIE**
**F-92415 Courbevoie Cédex (FR)**

(72) Inventeurs:
- **Billington, David C.**
  **Aston Triangle, Birmingham, B4 7ET (GB)**
- **Picard, Isabelle**
  **75015 Paris (FR)**
- **Atassi, Ghanem**
  **92210 Saint-Cloud (FR)**
- **Pierre, Alain**
  **78160 Marly le Roi (FR)**
- **Burbridge, Michael**
  **92400 Courbevoie (FR)**
- **Guilbaud, Nicolas**
  **78170 La Celle Saint Cloud (FR)**

## (54) Nouveaux composés cyclohexaniques, leur procédé de préparation et les compositions pharmaceutiques qui les contiennent

(57) Composés de formule (I):

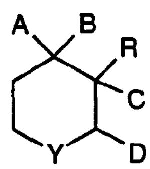

(I)

dans laquelle:

- **R** est choisi parmi les radicaux:

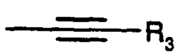 , 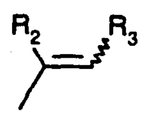 , 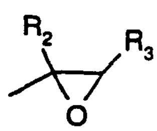 , et 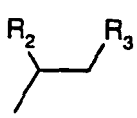 ,

(α) (β) (γ) (δ)

- **Y** est choisi parmi

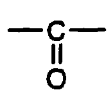 et 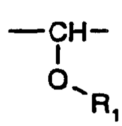

EP 0 739 887 A1

- **$R_1$** représente le radical

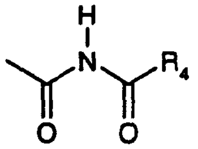

et A, B, C, D, $R_2$, $R_3$ et $R_4$ sont tels que définis dans la description, ainsi que leurs éventuels isomères géométriques et/ou diastéréoisomères et/ou énantiomères sous forme pure ou sous forme de mélange.

Médicaments et usage comme inhibiteur d'angiogenèse.

## Description

La présente invention conceme de nouveaux composés cyclohexaniques, leur procédé de préparation et les compositions pharmaceutiques qui les contiennent. Les composés de la présente invention trouvent une utilisation thérapeutique tout à fait intéressante grâce à leur pouvoir inhibiteur d'angiogenèse.

L'angiogenèse (ou néovascularisation) est définie comme le développement et la croissance de nouveaux vaisseaux sanguins capillaires. Le processus d'angiogenèse est essentiel dans de nombreuses situations physiologiques dont le développement de l'embryon, la cicatrisation normale de blessures et le développement de l'endometrium après menstruation. En dehors de ces situations, l'angiogenèse chez l'adulte normal est très rare et la mitose des cellules endothéliales qui génère les parois des vaisseaux sanguins est très lente, avec des temps de renouvellement cellulaire mesurés en années.

Une angiogenèse anormale (c'est-à-dire la stimulation de la croissance de nouveaux vaisseaux sanguins due à un syndrome pathologique) est une caractéristique établie pour de nombreuses maladies, notamment la rétinopathie diabétique, l'arthrite rhumatoïde, les hémangiomes et la croissance de tumeurs solides. L'angiogenèse peut également jouer un rôle important dans d'autres maladies comme la maladie artério-coronaire.

Dans le domaine de l'oncologie, il a été montré que la croissance de tumeurs solides est tout à fait dépendante du développement constant de nouveaux vaisseaux sanguins et qu'il est corrélé, pour les métastases de certains cancers, avec la taille croissante de la tumeur primaire. (J. Folkman, *New Engl. Med.,* 285 (1971), 1182-1185).

Un traitement pharmaceutique (c'est-à-dire à l'aide d'un inhibiteur d'angiogenèse) peut donc stopper la croissance de tumeurs primaires, empêcher ou réduire la formation de métastases, empêcher l'apparition de croissances secondaires. De tels inhibiteurs d'angiogenèse sont également utiles dans le traitement de maladies non-néoplasiques mentionnées précédemment dans lesquelles apparaît une activité angiogénique.

Des inhibiteurs d'angiogenèse sont décrits dans la littérature. L'art antérieur des composés de la présente invention est notamment illustré par les brevets EP-A-357061 et JP-A-01-233275 décrivant respectivement des dérivés de fumagillol (formule (A)) et d'ovalicine (formule (B)) :

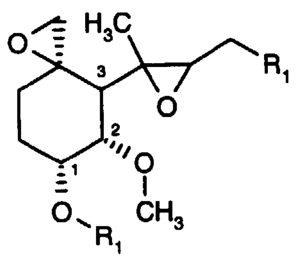

(A)

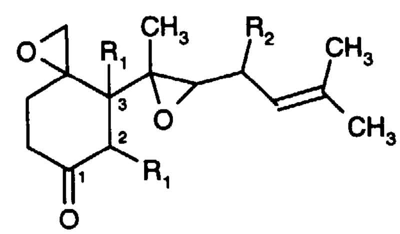

(B)

Les composés de la présente invention se différencient de ceux de l'art antérieur en ce qu'ils sont obtenus de manière entièrement synthétique permettant ainsi le contrôle total des centres d'asymétrie et l'accès à des molécules totalement originales grâce à des susbstituants sur l'atome de carbone 2 différent du groupement méthoxy ainsi que des groupements portés par l'atome de carbone 3 différents de ceux décrits dans les formules (A) et (B) décrites ci-dessus.

La recherche de structures originales a été guidée par le fait que les besoins de la thérapeutique exigent le développement constant de nouveaux composés inhibiteurs d'angiogenèse dans le but d'obtenir des principes actifs à la fois plus actifs, plus spécifiques et moins toxiques.

Plus particulièrement, la présente invention a pour objet les composés de formule générale (I) :

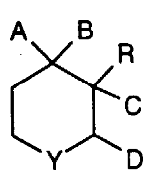

(I)

dans laquelle :

- **R** est choisi parmi les radicaux :

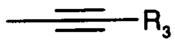 , 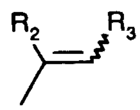 , 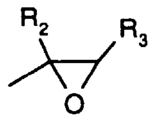 , et 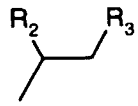 ,

(α) (β) (γ) (δ)

- **A** et **B** sont tels que :

  **A** représente le radical méthyle, et **B** représente le radical $-OR_1$,

  ou bien,

  et seulement dans le cas où R représente le radical (δ), **A** représente l'hydrogène et **B** est choisi parmi le radical $-CH_2-OH$ et le radical $-CH_2-OR_1$,

  ou bien,

  **A** représente le radical

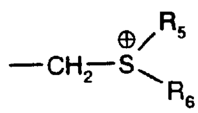

et **B** est choisi parmi le radical hydroxy, et le radical $-OR_1$,

  ou bien,

  **A** et **B** forment ensemble avec l'atome de carbone qui les porte et le groupement $-CH_2-O-$, un cycle oxirane,

- **C** et **D** sont tels que :

  **C** représente le radical hydroxy et **D** est choisi parmi l'hydrogène et le brome, ou bien,

  et seulement dans le cas où R représente le radical (δ), **C** représente le radical $OR_1$ et **D** est choisi parmi l'hydrogène et le brome,

  ou bien,

  et seulement dans le cas où R représente le radical (δ), **C** et **D** représentent chacun simultanément l'hydrogène,

  ou bien,

  **C** et **D** forment ensemble avec les atomes de carbone qui les portent et avec le groupement -O-, un cycle oxirane, ou bien forment ensemble avec les atomes de carbone qui les portent et le groupement -O-CO-O-, un hétérocycle dioxygéné, ou bien forment ensemble une liaison,

- **Y** est choisi parmi le radical -CO- et le radical $-CH(OR_1)-$,
- $\mathbf{R_1}$ représente le radical

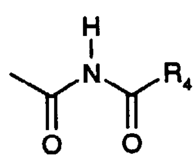 ,

- $\mathbf{R_2}$ est choisi parmi l'hydrogène, un radical alkyle éventuellement substitué, et un radical aryle éventuellement susbtitué,
- $\mathbf{R_3}$ est choisi parmi l'hydrogène, un radical alkyle éventuellement substitué et un radical arylalkyle éventuellement substitué,
- $\mathbf{R_4}$ est choisi parmi un radical alkyle éventuellement substitué, le radical amino, un radical alkylamino éventuellement substitué, un radical dialkylamino éventuellement substitué, un radical aryle éventuellement substitué, un radical arylalkyle éventuellement substitué, un radical hétéroarylalkyle éventuellement substitué, et un radical alkényle éventuellement substitué,
- $\mathbf{R_5}$ représente un radical alkyle éventuellement substitué,

- **$R_6$** est choisi parmi un radical alkyle éventuellement substitué, un radical aryle éventuellement substitué, un radical arylalkyle éventuellement substitué, un radical hétéroaryle éventuellement substitué et un radical hétéroarylalkyle éventuellement substitué,

étant entendu que :

- la chaîne alkyle dans les termes « alkyle » , « alkylamino », « dialkylamino », « arylalkyle » et « hétéroarylalkyle » désigne une chaîne hydrocarbonée saturée contenant de 1 à 6 atomes de carbone en chaîne droite ou ramifiée,
- le terme « aryle » désigne un radical choisi parmi phényle et naphtyle,
- le terme « hétéroaryle » désigne un radical choisi parmi pyridyle, quinolyle, isoquinolyle, imidazolyle, indolyle et isoindolyle,
- le terme « alkényle » désigne un radical choisi parmi vinyle et isopropényle,
- le terme « éventuellement substitué » associé aux radicaux alkyle, alkylamino, dialkylamino, aryle, arylalkyle, hétéroaryle, hétéroarylalkyle et alkényle, signifie que ces radicaux peuvent être éventuellement substitués, sur les parties acycliques et/ou, le cas échéant, sur les parties cycliques, par une ou plusieurs entités chimiques choisies parmi :
  - hydroxy,
  - halogène, choisi parmi fluor, chlore, brome et iode,
  - trihalogénométhyle,
  - nitro,
  - amino, alkylamino, dialkylamino
  - alkoxy, linéaire ou ramifié comportant de 1 à 6 atomes de carbone,
  - carboxy,
  - alkoxycarbonyle, linéaire ou ramifié comportant de 1 à 6 atomes de carbone, et,
  - acyle, linéaire ou ramifié comportant de 1 à 6 atomes de carbone,

  ainsi que leurs éventuels isomères géométriques, leurs éventuels diastéréoisomères et leurs éventuels énantiomères sous forme pure ou sous forme de mélange.

La présente invention s'étend également au procédé de préparation des composés de formule (I), caractérisé en ce que le composé de formule (II) :

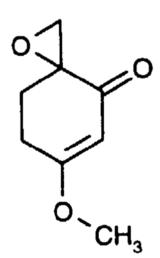

(II)

obtenu selon le mode opératoire décrit par E.J. Corey et J.P. Dittami, *J.A.C.S.,* (1985), *107,* 256-257 est soumis, selon le composé de formule (I) souhaité, à la combinaison d'une ou plusieurs des réactions suivantes :

***a) Pour l'insertion du radical R tel que défini dans la formule (I),***
par addition, en présence de butyl-lithium, en solvant polaire anhydre, tel que le toluène ou l'éther, seuls ou en mélange, sous atmosphère inerte, à température comprise entre -78°C et 0°C, d'un composé de formule (III) ou d'un composé de formule (IV) :

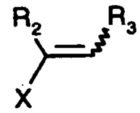

(III)

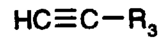

(IV)

dans lesquelles $R_2$ et $R_3$ sont tels que définis dans la formule (I) et X représente un atome d'halogène afin d'obtenir respectivement les composés pour lesquels R représente respectivement (β) ou (α),
les composés pour lesquels R représente (α) ou (β) définis précédemment pouvant être soumis à hydrogénation catalytique, pour conduire aux composés pour lesquels R représente (δ) tel que défini dans la formule (I),
les composés pour lesquels R représente (α) défini dans la formule (I) pouvant être soumis à l'action d'un agent oxydant, tel que l'acide métachloroperbenzoïque ou le diméthyldioxirane, de façon à obtenir les composés pour lesquels R représente (γ) défini dans la formule (I),

***b) Pour le radical Y défini dans la formule (I),***
par transformation de la fonction méthoxy du composé de formule (II) en fonction cétone lors de l'insertion du radical R décrite ci-dessus,
les composés pour lesquels Y représente -CO- pouvant, si on le souhaite, être soumis à l'action d'un réducteur, tel que le borohydrure de sodium pour obtenir l'alcool correspondant qui est soumis à l'action d'un isocyanate de formule (V) :

$$R_4\text{-CONCO} \qquad (V)$$

dans laquelle $R_4$ est tel que défini dans la formule (I),
de manière à obtenir les composés pour lesquels Y représente $-CH(OR_1)-$, $R_1$ étant tel que défini dans la formule (I),

***c) Pour les différentes valeurs de A et B,***

- pour les composés de formule (I) pour lesquels A et B forment ensemble avec l'atome de carbone qui les porte et le groupement $-CH_2-O-$, un cycle oxirane, sans aucune transformation, ce cycle oxirane étant déjà présent dans le composé de départ de formule (II) défini ci-dessus,

- pour les composés de formule (I) pour lesquels A représente le radical méthyle et B représente le radical $-OR_1$, par réaction du cycle oxirane présent dans le composé de formule (II) précédement défini, selon la réaction suivante :

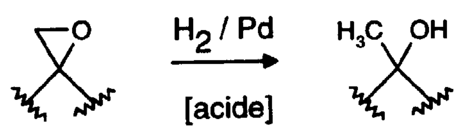


- pour les composés de formule (I) pour lesquels R représente le radical (δ) défini précédemment, A représente l'hydrogène et B est choisi parmi le radical $-CH_2-OH$ et le radical $-CH_2-OR_1$, par réaction du cycle oxirane présent dans la formule (II) précédemment définie, selon les réactions suivantes :

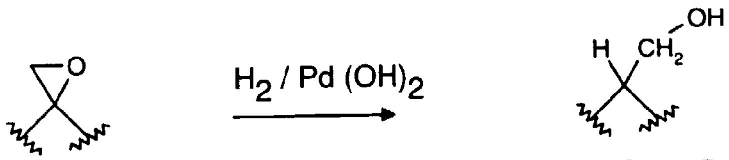


(VI)

(VI) + $R_4$-CONCO 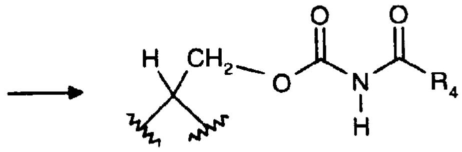

où $R_4$ est tel que défini dans la formule (I),

- pour les composés de formule (I) pour lesquels A représente le radical

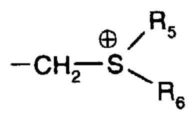

et B est choisi parmi le radical hydroxy et le radical $-OR_1$, par réaction du cycle oxirane présent dans la formule (II) précédemment définie selon les réactions suivantes :

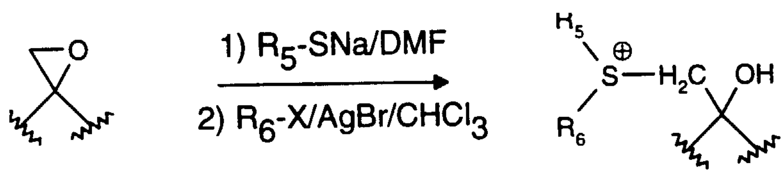


(VII)

(VII) + $R_4$-CONCO 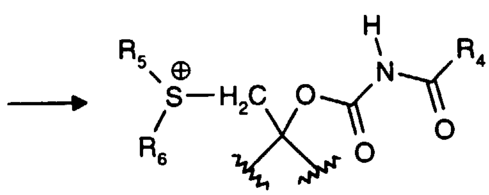

où $R_4$, $R_5$ et $R_6$ sont tels que définis dans la formule (I), et X représente un atome d'halogène,

***d) Pour les différentes valeurs de C et D,***

- pour les composés de formule (I) pour lesquels C représente le radical hydroxy et D représente le brome, par réaction de l'intermédiaire de synthèse de formule (VIII) obtenu lors de l'insertion du radical R (voir paragraphe a) ci-dessus) avec un agent de bromation, tel que le N-bromosuccinimide, selon la réaction suivante :

(VIII) 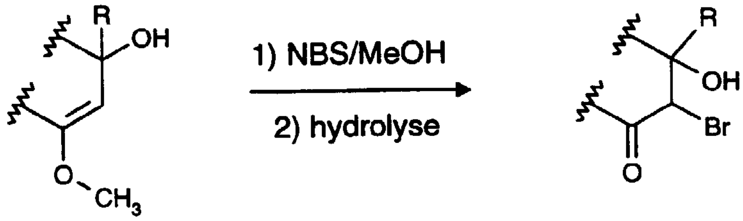


(IX)

où R est tel que défini précédemment,

- pour les composés de formule (I) pour lesquels C représente le radical hydroxy et D représente l'hydrogène, par réaction de l'intermédiaire de synthèse de formule (VIII) défini précédemment, selon la réaction suivante :

(VIII) 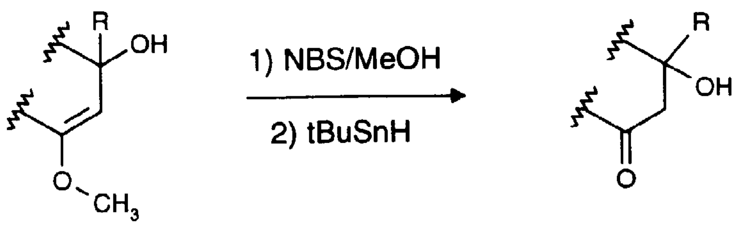

(X)

où R est tel que défini précédemment,
les fonctions hydroxy des composés de formules (IX) et (X) définis ci-dessus pouvant être carbamoylées sous l'action de l'isocyanate de formule $R_4$-CONCO, $R_4$ étant tel que défini précédemment, de façon à obtenir les composés de formule (I) pour lesquels D est choisi parmi l'hydrogène et le brome et C représente le radical $OR_1$

- les composés de formule (I) pour lesquels les valeurs de C et D étant autres que celles définies ci-dessus étant obtenus à partir du composé de formule (VIII) selon le schéma suivant :

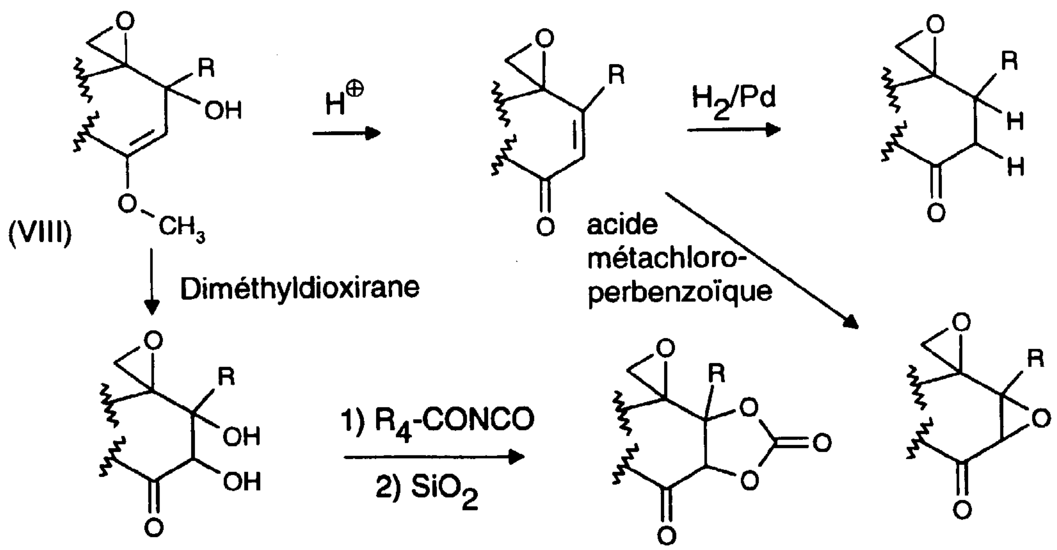


où R et $R_4$ sont tels que définis précédemment,
l'ensemble des intermédiaires de synthèse ainsi que les composés de formule (I) pouvant éventuellement être purifiés par une technique classique de purification et séparés si on le désire et le cas échéant en isomères géométriques et/ou diastéréoisomères et/ou énantiomères par une technique classique de séparation.

Les composés de formule (I) pour lesquels C et D forment ensemble, avec les atomes de carbone qui les portent et avec le groupement -O-, un cycle oxirane peuvent également être obtenus selon la réaction suivante :

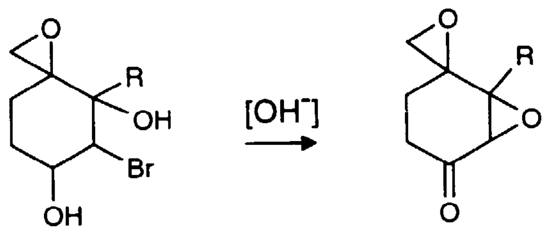

où R est tel que défini précédement.

Les composés de formule (I) pour lesquels R représente le radical (δ) tel que défini précédemment peuvent avantageusement être obtenu par action, sur le composé de formule (II) précédemment défini, d'un composé de formule (XI) :

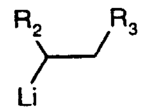

(XI)

où $R_2$ et $R_3$ sont tels que définis précédemment,
en présence de chlorure de cérium(III), dans le tétrahydrofurane, à température appropriée, par exemple -78°C.

Les différentes réactions décrites dans le procédé exposé précédemment seront réalisés dans un ordre chimiquement convenable déterminé par l'homme du métier à la lumière des exemples décrits plus loin dans la description. De manière générale, le radical R sera introduit en début de synthèse, et les réactions de carbomoylation seront réalisées au cours des dernières étapes de synthèse.

Les composés de formule (I) possèdent d'intéressantes propriétés pharmacologiques. En effet, ces composés sont de puissants inhibiteurs d'angiogenèse qui ont l'avantage de présenter, par rapport aux composés de référence, une toxicité beaucoup moins importante. Ils présentent donc un index thérapeutique excellent.

Ces composés trouvent ainsi une application en thérapeutique en tant qu'agents anti-tumoraux, dans l'inhibition de la formation et de la croissance des métastases, ainsi que dans le traitement de la rétinopathie diabétique, de l'arthrite rhumatoïde, des hémangiomes et des maladies artério-coronaires, et plus généralement dans les affections dues ou reliées aux troubles de l'angiogenèse.

La présente invention a également pour objet les compositions pharmaceutiques contenant les composés de formule (I), leurs éventuels isomères géométriques et/ou diastéréoisomères et/ou énantiomères sous forme pure ou sous forme de mélange, seuls ou en combinaison avec un ou plusieurs excipients ou véhicules inertes et non toxiques.

Parmi les compositions pharmaceutiques selon l'invention, il sera cité plus particulièrement celles qui conviennent pour l'administration orale, parentérale, nasale, rectale, perlinguale, transdermique, oculaire ou respiratoire, et notamment les comprimés simples ou dragéifiés, les comprimés sublinguaux, les gélules, les suppositoires, les crèmes, les pommades, les gels dermiques, les patches, les préparations injectables ou buvables, les aérosols, les gouttes oculaires ou nasales.

La posologie utile varie selon l'âge et le poids du patient, la voie d'administration, la nature de l'indication thérapeutique et des traitements éventuels associés et s'échelonne entre 0,01 et 1 g par jour, en une ou plusieurs administrations.

Les exemples suivants illustrent l'invention mais ne la limitent en aucune façon. Les produits de départ sont connus ou préparés à partir de modes opératoires connus.

**<u>EXEMPLE 1</u> : (3*S**, 4*S**, 5*R**, 6*R**)-5-Bromo-6-chloroacétylcarbamoyloxy-4-hydroxy-4-(1-méthylpropén-1-yl)-1-oxa-*spiro*[2.5]octane**

(±)

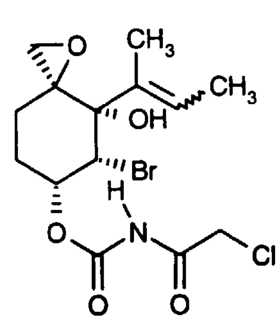

<u>Etape A</u> : 4-hydroxy-6-méthoxy-4-(1-méthylpropén-1-yl)-1-oxa-*spiro*[2.5]oct-5-ène

A une solution de 4,62 ml de 2-bromo-2-butène *(cis, trans)* (45,41 mmol) dans 140 ml de diéthyléther anhydre, refroidie à -78°C, sont ajoutés goutte-à-goutte et sous atmosphère d'azote 54 ml de *tert*-butyllithium (1,7 M dans le

pentane soit 91,89 mmol). L'ensemble est laissé sous agitation à -78°C pendant 5 minutes puis à 0°C pendant 1 heure. A cette solution est alors additionnée goutte-à-goutte une solution de 6-méthoxy-1-oxa-*spiro*[2.5]oct-5-én-4-one, préparé suivant le mode opératoire décrit par E. J. Corey et J. P. Dittami, (*J. A. C. S.* (1985), *107,* 256-257) (4 g ; 25,94 mmol) dans 40 ml de toluène anhydre refroidie à -78°C. Après 1 heure d'agitation à -78°C, le mélange réactionnel est versé dans une solution aqueuse à 10% de chlorure d'ammonium (400 ml). L'ensemble est dilué à l'éther éthylique (400 ml) et le mélange réactionnel est extrait. Le traitement habituel de la phase organique foumit un résidu huileux qui est chromatographié sur gel de silice (éluant : pentane/acétate d'éthyle, 7:1), pour obtenir 3,4 g (16,17 mmol) de produit attendu.
Rendement : 60%

Etape B : 5-bromo-6,6-diméthoxy-4-hydroxy-4-(1-méthylpropén-1-yl)-1-oxa-*spiro*[2.5]octane

A une solution de 3,4 g (16,17 mmol) du composé obtenu à l'étape A dans 160 ml de méthanol refroidie à 0°C sont ajoutés 3,4 g (19,32 mmol) de N-bromosuccinimide. Après 15 minutes d'agitation à 0°C, le mélange réactionnel est évaporé. Le résidu huileux est repris par de l'acétate d'éthyle. Après traitement de la phase organique et purification sur gel de silice (éluant : pentane/acétate d'éthyle 8:1), 3,2 g (9,96 mmol) de produit attendu sont obtenus.
Rendement : 62%

Etape C : 5-bromo-4-hydroxy-4-(1-méthylpropén-1-yl)-1-oxa-*spiro*[2.5]octan-6-one

A une solution de 3,2 g (9,96 mmol) du composé obtenu à l'étape B dans 120 ml d'un mélange acétone/eau (3:2) à température ambiante sont ajoutés 1,9 g d'acide paratoluènesulfonique (9,96 mmol). Lorsque la réaction, suivie par chromatographie sur couche mince, est terminée, l'acétone est alors évaporé. La phase aqueuse restante est ensuite extraite avec de l'acétate d'éthyle (200 ml). Après traitement habituel de la phase organique, 2,23 g (8,1 mmol) de produit attendu sont obtenus.
Rendement : 81%

Etape D : 5-bromo-4,6-hydroxy-4-(1-méthylpropén-1-yl)-1-oxa-*spiro*[2.5]octane

A une solution de 1 g (3,63 mmol) du composé obtenu à l'étape C dans 110 ml d'un mélange dichlorométhane/méthanol (10:1) à température ambiante sont ajoutés par petites portions 137 mg (3,6 mmol) de borohydrure de sodium. Lorsque la réaction, suivie par chromatographie sur couche mince, est terminée, le milieu réactionnel est concentré, repris par du méthanol, puis reconcentré. Après chromatographie sur gel de silice (éluant : pentane/acétate d'éthyle, 4:1), 0,46 g (1,66 mmol) de produit attendu sont obtenus.
Rendement : 46%

Etape E : (3*S**, 4*S**, 5*S**, 6*R**)-5-bromo-6-chloroacétylcarbamoyloxy-4-hydroxy-4-(1-méthylpropén-1-yl)-1-oxa-*spiro* [2. 5]octane

A une solution de 0,46 g (1,66 mmol) du composé obtenu à l'étape D dans 10 ml de dichlorométhane anhydre refroidie à 0°C sont ajoutés, goutte-à-goutte et sous atmosphère d'azote, 0,26 ml (3,087 mmol) d'isocyanate de chloroacétyle. Après 30 minutes d'agitation à 0°C, on verse 10 ml d'eau au mélange réactionnel et l'ensemble est laissé sous agitation pendant 1 heure. Après traitement habituel de la phase organique, 0,46 g (1,16 mmol) du composé attendu sont obtenus sous forme de mousse.
Rendement : 70%

**<u>EXEMPLE 2</u> : (3*S**, 4*R**, 5*R**, 6*R**)-5-Bromo-6-chloroacétylcarbamoyloxy-4-hydroxy-4-(2,3-diméthyloxiran-2-yl)-1-oxa-*spiro*[2.5]octane**

(±) 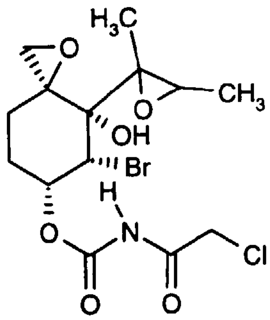

A une solution de 0,46 g (1,16 mmol) du composé obtenu à l'exemple 1 dans 30 ml de dichlorométhane sont ajoutés 1,12 g (4,74 mmol) d'acide 3-chloroperbenzoïque à 72%. Le mélange réactionnel est placé sous agitation à température ambiante pendant 5 heures. Le solide formé en cours de réaction est filtré. Après traitement habituel de la phase organique et deux purifications par chromatographie sur gel de silice (éluant : hexane/acétate d'éthyle, 3:2), 0,146 mg (0,354 mmol) du composé attendu sont obtenus sous forme de mousse.
<u>Rendement</u> : 48%

<u>Analyse élémentaire</u> :

| (Formule brute : $C_{14}H_{19}BrClNO_6$ masse moléculaire : 412,66) | | | | | |
|---|---|---|---|---|---|
| | **C** | **H** | **N** | **Br** | **Cl** |
| **% trouvé** | 40,76 | 4,78 | 3,42 | 19,00 | 8,00 |
| **% calculé** | 40,75 | 4,64 | 3,39 | 19,36 | 8,60 |

**<u>EXEMPLE 3</u> : (3*S**, 4*S**, 5*R**, 6*R**)-5-Bromo-6-chloroacétylcarbamoyloxy-4-hydroxy-4-(propén-2-yl)-1-oxa-*spiro*[2.5]octane**

(±) 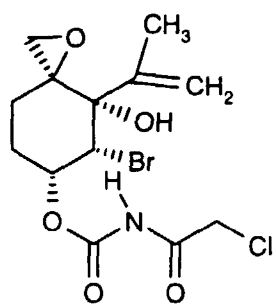

Etape A : 4-hydroxy-6-méthoxy-4-(propén-2-yl)-1-oxa-*spiro*[2.5]-5- oct-5-ène

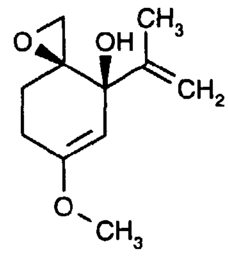

Isomère A

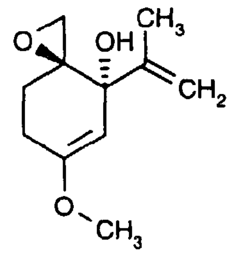

Isomère B

En procédant selon le mode opératoire décrit à l'étape A de l'exemple 1, à partir de 9,2 ml (0,104 mol) de 2-bromo-1-propène, de 115 ml de *tert*-butyllithium (1,7 M dans le pentane soit 0,196 mol) dans 335 ml de diéthyléther anhydre et de 7,88 g (51,2 mmol) de 6-méthoxy-1-oxa-*spiro*[2.5]oct-5-ène-4-one dans 80 ml de toluène anhydre, 0,94 g (4,79 mmol) d'isomère A et 5,82 g (19,24 mmol) d'isomère B sont obtenus après chromatographie sur gel de silice (éluant : pentane/acétate d'éthyle; 7:1).
Rendement : 67%

Etape B : 5-bromo-6,6-diméthoxy-4-hydroxy-4-(propén-2-yl)-1-oxa-*spiro*[2.5] octane

En procédant selon le mode opératoire décrit à l'étape B de l'exemple 1, à partir de 8,37g (42,67 mmol) d'isomère B obtenu à l'étape A et de 8,25 g (46,97 mmol) de N-bromosuccinimide dans 420 ml de méthanol, 10,01 g (32,52 mmol) de composé attendu sont obtenus après deux chromatographies sur gel de silice (éluant : éther de pétrole/acétate d'éthyle, 9:1).
Rendement : 76%

Etape C : 5-bromo-4-hydroxy-4-(propén-2-yl)-1-oxa-*spiro*[2.5]octan-6-one

En procédant selon le mode opératoire décrit à l'étape C de l'exemple 1, à partir de 4,58 g (14,89 mmol) de composé obtenu à l'étape B et de 4,25 g (22,33 mmol) d'acide paratoluènesulfonique dans 400 ml d'un mélange acétone/eau (3:2), 3,54 g (13,56 mmol) de composé attendu sont obtenus.
Rendement : 91%

Etape D : 5-bromo-4,6-hydroxy-4-(propén-2-yl)-1-oxa-*spiro*[2.5]octane

En procédant selon le mode opératoire décrit à l'étape D de l'exemple 1, à partir de 4,34 g (16 mmol) de composé obtenu à l'étape C et de 0,64 g (16 mmol) de borohydrure de sodium dans 550 ml d'un mélange dichlorométhane/méthanol (10:1), 3,2 g (12,16 mmol) de composé attendu sont obtenus.
Rendement :73%

Etape E : (3*S**, 4*S**, 5*R**, 6*R**)-5-bromo-6-chloroacétylcarbamoyloxy-4-hydroxy-4-(propén-2-yl)-1-oxa-*spiro*[2.5]octane

En procédant selon le mode opératoire décrit à l'étape E de l'exemple 1, à partir de 1,03 g (3,9 mmol) de composé obtenu à l'étape D et de 0,62 ml (7,3 mmol) d'isocyanate de chloroacétyle dans 20 ml de dichlorométhane anhydre, 2,14 g (5,6 mmol) de composé attendu sont obtenus.

**EXEMPLE 4 : (3*S**, 4*S**, 5*R**, 6*R**)-5-Bromo-6-chloroacétylcarbamoyloxy-4-hydroxy-4-2-(méthyl-2-oxiranyl)-1-oxa-*spiro*[2.5]octane**

(±)

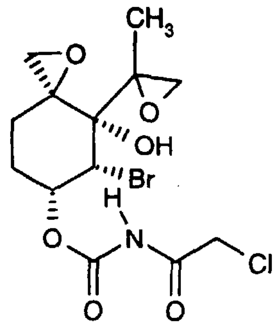

En procédant selon le mode opératoire décrit à l'exemple 2, à partir de 2,14 g (5,6 mmol) de composé brut obtenu à l'exemple 3 et de 1,93 g (11,2 mmol) d'acide 3-chloroperbenzoïque à 72% dans 55 ml de dichlorométhane, 0,9 g (2,26mmol) d'un mélange de deux diastéréoisomères attendus (73/27) sont obtenus après deux chromatographies sur gel de silice (éluant : hexane/acétate d'éthyle, 3:1), sous forme de mousse.
Rendement : 84%

Analyse élémentaire :

| (Formule brute : $C_{13}H_{17}BrClNO_6$ masse moléculaire : 398,64) | | | | | |
|---|---|---|---|---|---|
| | **C** | **H** | **N** | **Br** | **Cl** |
| **% trouvé** | 40,30 | 4,20 | 3,67 | 19,00 | 9,50 |
| **% calculé** | 39,17 | 4,30 | 3,51 | 20,04 | 8,89 |

**EXEMPLE 5 : (3*S**, 4*S**, 6*R**)-6-Chloroacétylcarbamoyloxy-4-hydroxy-4-(1-méthyl-propén-1-yl)-1-oxa-*spiro*[2.5] octane**

(±)

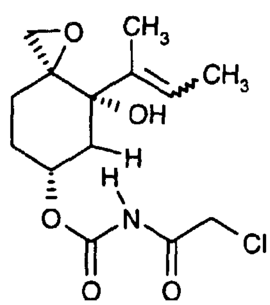

Etape A : 4,6-hydroxy-4-(1-méthylpropén-1-yl)-1-oxa-*spiro*[2.5]octane

A une solution de 0,94 g (3,39 mmol) de composé obtenu dans l'exemple 1 à l'étape D dans 35 ml de toluène anhydre sont ajoutés 1,8 ml (6,78 mmol) d'hydrure de tributylétain. Le mélange réactionnel est placé sous agitation et chauffé à reflux. Lorsque la réaction, suivie par chromatographie sur couche mince, est terminée, le mélange est refroidi puis évaporé. Le résidu huileux est repris par de l'acétate d'éthyle et 3 g de fluorure de potassium dans 15 ml d'eau sont additionnés. Après 15 minutes d'agitation, le fluorure de tributylétain formé est filtré. Après traitement habituel de la phase organique et purification par chromatographie sur gel de silice (éluant : pentane/acétate d'éthyle, 2:1), 0,32 g (1,61 mmol) du composé *trans* et 0,31 g (1,56 mmol) du composé *cis* attendus sont obtenus.
Rendement : 94%

Etape B : (3*S**, 4*S**, 6*R**)-6-chloroacétylcarbamoyloxy-4-hydroxy-4-(1-méthyl-propén-1-yl)-1-oxa-*spiro*[2.5]octane.

En procédant selon le mode opératoire décrit à l'étape E de l'exemple 1, à partir de 0,32 g (1,61 mmol) de composé *trans* obtenu à l'étape A et de 0,245 ml (2,91 mmol) d'isocyanate de chloroacétyle dans 10 ml de dichlorométhane anhydre, 0,5 g (1,57 mmol) de composé attendu sont obtenus.
Rendement : 97%

**EXEMPLE 6 : (3*S**, 4*R**, 6*R**)-6-Chloroacétylcarbamoyloxy-4-hydroxy-4-(2,3-diméthyloxiran-2-yl)-1-oxa-*spiro* [2.5]octane (isomère *trans*)**

(±)

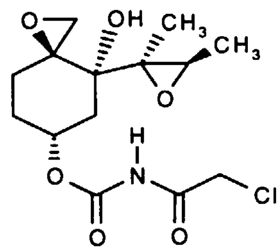

En procédant selon le mode opératoire décrit à l'exemple 2, à partir de 0,5 g (1,57 mmol) de composé obtenu à l'exemple 5 et de 0,38 g (3,14 mmol) d'acide 3-chloroperbenzoïque à 72% dans 25 ml de dichlorométhane, 0,33 g (0,99 mmol) de composé attendu sont obtenus après chromatographie sur gel de silice (éluant : heptane/acétate d'éthyle, 1:1).
Rendement : 63%

Analyse élémentaire :

| (Formule brute : $C_{14}H_{20}ClNO_6$ masse moléculaire : 333,77) | | | | |
|---|---|---|---|---|
| | **C** | **H** | **N** | **Cl** |
| **% trouvé** | 50,88 | 6,45 | 4,01 | 10,45 |
| **% calculé** | 50,38 | 6,04 | 4,20 | 10,62 |

**EXEMPLE 7 : (3*S**, 4*R**, 6*R**)-6-Chloroacétylcarbamoyloxy-4-hydroxy-4-(2,3-di-méthyl-2-oxiranyl)-1-oxa-*spiro* [2-5]octane (isomère *cis)***

(±)

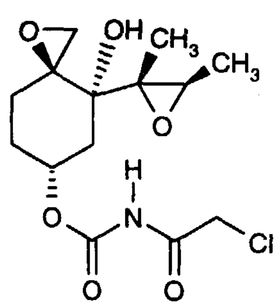

En procédant selon le mode opératoire décrit à l'exemple 6 à partir de l'isomère *cis* obtenu à l'étape A de l'exemple 5, traité selon le mode opératoire décrit à l'étape B de l'exemple 5, 0,145 g (0,43 mmol) de composé attendu sont obtenus.
Rendement : 64%

Analyse élémentaire :

| (Formule brute : $C_{14}H_{20}ClNO_6$ masse moléculaire 333,77) | | | | |
|---|---|---|---|---|
| | **C** | **H** | **N** | **Cl** |
| **% trouvé** | 50,73 | 6,40 | 4,13 | 10,76 |
| **% calculé** | 50,38 | 6,04 | 4,20 | 10,62 |

**EXEMPLE 8 : (3*S**, 4*S**, 6*R**)-6-Chloroacétylcarbamoyloxy-4-hydroxy-4-(propén-2-yl)-1-oxa-*spiro*[2.5]octane**

(±) 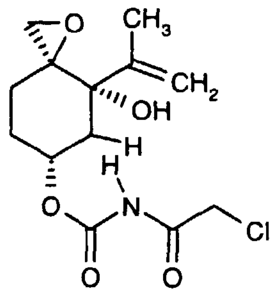

Etape A : 4,6-hydroxy-4-(propén-2-yl)-1-oxa-*spiro*[2.5]octane

En procédant selon le mode opératoire décrit à l'étape A de l'exemple 5, à partir de 2,16 g (8,2 mmol) du composé obtenu à l'étape D de l'exemple 3 et de 4,4 ml (16,4 mmol) d'hydrure de tributylétain dans 85 ml de toluène anhydre, 1,74 g (9,44 mmol) de composé brut attendu sont obtenus.

Etape B : (3*S**, 4*S**, 6*R**)-6-Chloroacétylcarbamoyloxy-4-hydroxy-4-(propén-2-yl)-1-oxa-*spiro*[2.5]octane

En procédant selon le mode opératoire décrit à l'étape E de l'exemple 1, à partir de 1,74 g (9,44 mmol) de composé brut obtenu à l'étape précédente et de 1,5 ml (17,6 mmol) d'isocyanate de chloroacétyle dans 45 ml de dichlorométhane anhydre, 1,87 g (6,15 mmol) de composé attendu sont obtenus.

**EXEMPLE 9 : (3*S**, 4*S**, 6*R**)-6-Chloroacétylcarbamoyloxy-4-hydroxy-4-(2-méthyloxiran-2-yl)-1-oxa-*spiro*[2.5] octane**

(±) 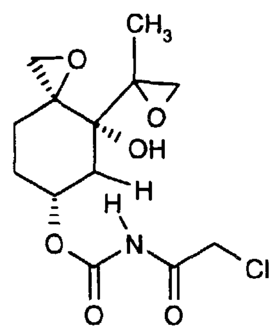

En procédant selon le mode opératoire décrit à l'exemple 2, à partir de 1,66 g (5,5 mmol) de composé obtenu dans l'exemple 8 et de 1,9 g (11,0 mmol) d'acide 3-chloro-perbenzoïque à 72% dans 60 ml de dichlorométhane, 1,4 g (4,38 mmol) de composé attendu sont obtenus après deux chromatographies sur gel de silice (éluant : hexane/acétate d'éthyle, 3:2) sous forme de mousse.
Rendement : 80%

Analyse élémentaire :

| (Formule brute : $C_{13}H_{18}ClNO_6$ masse moléculaire : 319,74) | | | | |
|---|---|---|---|---|
| | **C** | **H** | **N** | **Cl** |
| **% trouvé** | 48,73 | 5,79 | 4,42 | 11,53 |
| **% calculé** | 48,83 | 5,67 | 4,38 | 11,09 |

**EXEMPLE 10 : (3*S**, 4*S**, 5*R**, 6*R**)-5-Bromo-6-chloroacétylcarbamoyloxy-4-hydroxy-4-(5-phénylpentyl)-1-oxa-*spiro*[2.5]octane**

(±) 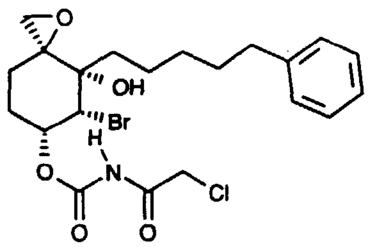

Etape A : 5-bromo-4-hydroxy-4-(5-phénylpentyl)-1-oxa-*spiro*[2.5]octan-6-one

A une solution de 25 ml (0,157 moles) de 5-phénylpentyne dans 400 ml de diéthyléther anhydre refroidie à -78°C sont ajoutés goutte-à-goutte et sous atmosphère d'azote 74 ml (0,118 moles) de n-butyllithium (1,6 molaire dans l'hexane). L'ensemble est laissé sous agitation à -78°C pendant 15 minutes puis à 0°C pendant 75 minutes. A cette solution est alors additionnée goutte-à-goutte une solution de 6-méthoxy-1-oxa-*spiro*[2.5]oct-5-én-4-one (11 g ; 71,33 mmol) dans 80 ml de toluène anhydre refroidie à -78°C. Après 1 heure d'agitation à -78°C, le mélange réactionnel est versé dans une solution aqueuse à 10 % de chlorure d'ammonium (1000 ml). Le traitement habituel de la phase organique fournit 22 g (74 mmol) de 4-hydroxy-6-méthoxy-4-(5-phénylpentén-1-yl)-1-oxa-*spiro*[2.5]octène (2 iso-mères) sous la forme d'un résidu huileux que l'on utlise sans purification pour l'étape suivante.
L'hydrogénation d'une solution de 22 g (74 mmol) de ce mélange d'isomères dans 610 ml de benzène en présence de 10 g de catalyseur de Lindlar pendant 7 heures conduit après filtration et évaporation à 22 g (73 mmol) de 4-hydroxy-6-méthoxy-4-(5-phénylpentyl)-1-oxa-*spiro*[2.5]oct-5-ène sous la forme d'un résidu huileux que l'on utilise sans purification pour l'étape suivante.
A une solution de 22 g (73 mmol) du mélange précédent dans 100 ml de méthanol, refroidie à 0°C, sont ajoutés 13,94 g (78,32 mmol) de N-bromosuccinimide. Après 30 minutes d'agitation à 0°C, le mélange réactionnel est évaporé. Le résidu huileux est repris par de l'acétate d'éthyle. Après traitement habituel de la phase organique et purification sur gel de silice (éluant : heptane/acétate d'éthyle, 3:1), 5 isomères sont obtenus dans les proportions suivantes : 8,62 g (20,85 mmol) d'isomère A, 5,19 g (12,55 mmol) d'isomère B, 6,89 g (16,67 mmol) d'un mélange d'isomères C et E et 3,1 g (7,5 mmol) d'isomère D.

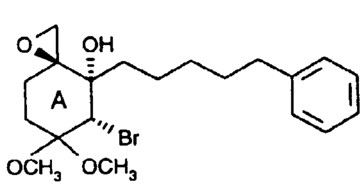

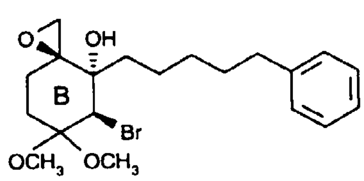

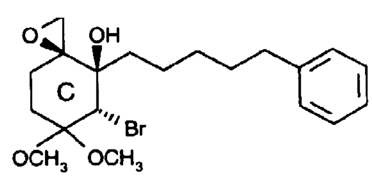

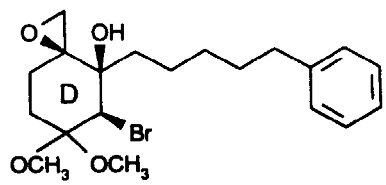

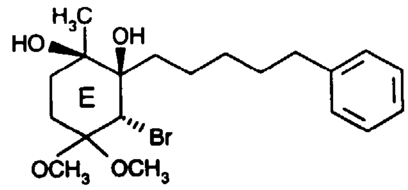

En procédant selon le mode opératoire décrit à l'étape C de l'exemple 1, à partir de 8,62 g (20,9 mmol) d'isomères A, B, C ou D et de 4 g (21 mmol) d'acide paratoluènesulfonique dans 250 ml d'un mélange acétone/eau (3:2), 5,06 g (13,78 mmol) de composé attendu sont obtenus.

Rendement : 66%

Etape B : 5-bromo-4,6-hydroxy-4-(5-phénylpentyl)-1-oxa-spiro[2.5]octane

En procédant selon le mode opératoire décrit à l'étape D de l'exemple 1, à partir de 5,06 g (13,78 mmol) de composé obtenu à l'étape précédente et de 0,29 g (7,66 mmol) de borohydrure de sodium dans 550 ml d'un mélange dichlorométhane/méthanol (10:1), 3,85 g (10,42 mmol) de composé attendu sont obtenus après chromatographie sur gel de silice (éluant : pentane/acétate d'éthyle, 4:1). Rendement : 76%

Etape C : 5-bromo-6-chloroacétylcarbamoyloxy-4-hydroxy-4-(5-phénylpentyl)-1-oxa-*spiro*[2.5]octane

En procédant selon le mode opératoire décrit à l'étape E de l'exemple 1, à partir de 1 g (2,7 mmol) de composé obtenu à l'étape précédente et de 0,42 ml (5,03 mmol) d'isocyanate de chloroacétyle dans 30 ml de dichlorométhane anhydre, 0,497 g (1,017 mmol) de composé attendu sont obtenus après chromatographie sur gel de silice (éluant : heptane/acétate d'éthyle, 5:2).

Rendement : 38%

Analyse élémentaire :

| (Formule brute : $CH_{2127}BrClNO_5$ masse moléculaire : 488,81) | | | | | |
|---|---|---|---|---|---|
| | **C** | **H** | **N** | **Cl** | **Br** |
| **% trouvé** | 53,07 | 5,58 | 2,93 | 6,00 | 8,00 |
| **% calculé** | 51,60 | 5,57 | 2,87 | 16,35 | 7,2 |

**EXEMPLE 11 : (3*S**, 4*R**, 6*S**)-6-Chloroacétylcarbamoyloxy-4-hydroxy-4-(5-phénylpentyl)-1-oxa-*spiro*[2.5]octane**

(±)

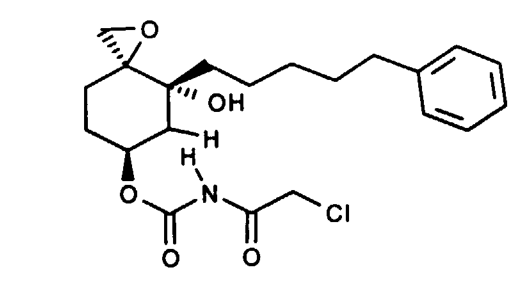

Etape A : (3*S**, 4*R**, 6*S**)-4,6-hydroxy-4-(5-phénylpentyl)-1-oxa-*spiro*[2.5]octane

En procédant selon le mode opératoire décrit à l'étape A de l'exemple 5, à partir de 1,02 g (2,77 mmol) de l'isomère C obtenu à l'étape B de l'exemple 10 et de 1,2 ml (4,52 mmol) d'hydrure de tributylétain dans 25 ml de toluène anhydre, 0,54 g (1,86 mmol) de composé attendu sont obtenus après chromatographie sur gel de silice (éluant : pentane/acétate

d'éthyle, 3:1).
Rendement : 67%

Etape B : (3*S**, 4*R**, 6*S**)-6-chloroacétylcarbamoyloxy-4-hydroxy-4-(5-phényl-pentyl)-1-oxa-*spiro*[2.5]octane

En procédant selon le mode opératoire décrit à l'étape E de l'exemple 1, à partir de 0,54 g (1,86 mmol) de composé obtenu à l'étape précédente et de 0,29 ml (3,5 mmol) d'isocyanate de chloroacétyle dans 18 ml de dichlorométhane anhydre, puis purification par chromatographie sur gel de silice (éluant : heptane/acétate d'éthyle, 2:1), on obtient 0,24 g (0,59 mmol) de composé attendu et 0,38 g (0,72 mmol) de composé de l'exemple 12.
Rendement : 32%

Analyse élémentaire :

| (Formule brute : $C_{21}H_{28}ClNO_5$ masse moléculaire : 409,91) | | | |
|---|---|---|---|
| | **C** | **H** | **N** |
| **% trouvé** | 62,79 | 7,18 | 3,32 |
| **% calculé** | 61,53 | 6,89 | 3,42 |

**EXEMPLE 12 : (3*S**, 4*R**, 6*S**)-4,6-Dichloroacétylcarbamoyloxy-4-(5-phénylpentyl)-1-oxa-*spiro*[2.5]octane**

(±)

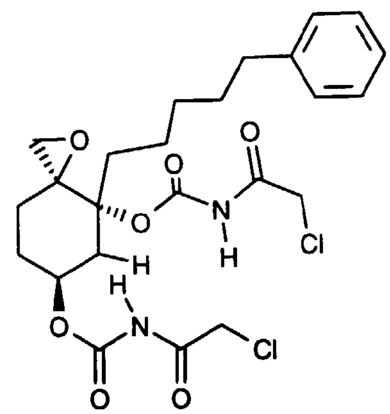

Ce composé dicarbamoylé est obtenu en suivant le mode opératoire décrit à l'exemple 11.
Rendement : 39 %

Analyse élémentaire :

| (Formule brute : $C_{24}H_{30}Cl_2N_2O_7$ masse moléculaire : 529,42) | | | |
|---|---|---|---|
| | **C** | **H** | **N** |
| **% trouvé** | 54,25 | 5,94 | 5,47 |
| **% calculé** | 54,45 | 5,71 | 5,29 |

**<u>EXEMPLE 13</u> : (3*S**, 4*R**, 5*S**, 6*S**)-5-Bromo-6-chloroacétylcarbamoyloxy-4-hydroxy-4-[3-(3-phénylpentyl)oxiran-2-yl]-1-oxa-*spiro*[2.5]octane**

(±) 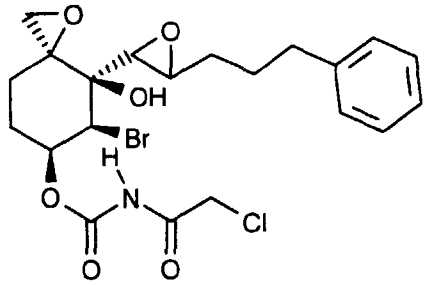

<u>Etape A</u> : 5-bromo-4-hydroxy-4-(5-phénylpentén-1-yl)-1-oxa- *spiro*[2.5]octan-6-one

L'hydrogénation d'une solution de 4-hydroxy-6-méthoxy-4-(5-phénylpentyn-1-yl)-1-oxa-*spiro*[2.5]octène, mélange brut d'isomères, obtenus selon le mode opératoire décrit à l'étape A de l'exemple 10 (10,67 g ; 35,52 mmol) dans 300 ml de benzène en présence de 5 g de catalyseur de Lindlar pendant 1 heure conduit après filtration et évaporation à 10,67 g (35,5 mmol) de 4-hydroxy-6-méthoxy-4-(5-phénylpentén-1-yl)-1-oxa-*spiro*[2.5]oct-5-ène (2 isomères) sous la forme d'un résidu huileux que l'on utilise sans purification pour l'étape suivante. A une solution de 10,67 g (35,5 mmol) du mélange précédent dans 360 ml de méthanol refroidie à 0°C sont ajoutés 7,58 g (42,6 mmol) de N-bromosuccinimide. Après 15 minutes d'agitation à 0°C, le mélange réactionnel est évaporé. Le résidu huileux est repris par de l'acétate d'éthyle. Après traitement de la phase organique à l'eau et purification par chromatographie sur gel de silice (éluant : heptane/acétate d'éthyle, 5:1), 2 isomères sont obtenus dans les proportions suivantes : 1,5 g (3,65 mmol) d'isomère A et 3,14 g (7,63 mmol) d'isomère B.

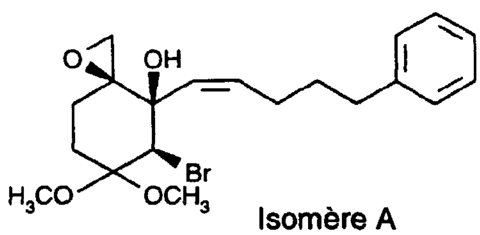

Isomère A

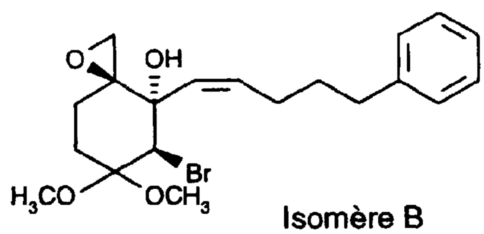

Isomère B

En procédant selon le mode opératoire décrit à l'étape C de l'exemple 1, à partir de 1,5 g (3,65 mmol) d'isomère A obtenu précédemment et de 0,84 g (4,4 mmol) d'acide paratoluènesulfonique dans 125 ml d'un mélange acétone/eau (3:2), 0,9 g (2,46 mmol) de composé attendu sont obtenus après chromatographie sur gel de silice (éluant : toluène/acétate d'éthyle, 9:1).
<u>Rendement</u> : 78%

<u>Etape B</u> : 5-bromo-4,6-hydroxy-4-(5-phénylpentén-1-yl)-1-oxa-*spiro*[2.5]-octane

En procédant selon le mode opératoire décrit à l'étape D de l'exemple 1, à partir de 0,9 g (2,46 mmol) de composé obtenu à l'étape A et de 45 mg (1,19 mmol) de borohydrure de sodium dans 100 ml d'un mélange dichlorométhane/méthanol (10:1), 0,26 g (0,71 mmol) de composé attendu sont obtenus après chromatographie sur gel de silice (éluant : pentane/acétate d'éthyle, 3:1).
<u>Rendement</u> : 21%

<u>Etape C</u> : 5-bromo-4,6-hydroxy-4-[3-(3-phénylpentyl)oxiran-2-yl]-1-oxa-*spiro*[2.5] octane

0,26 g (0,71 mmol) de composé obtenu à l'étape précédente sont mis en solution dans 30 ml de diméthyldioxirane refroidis à -20°C. Le mélange réactionnel est placé sous agitation pendant plusieurs heures de -20°C à température ambiante puis concentré. Le résidu huileux résultant est chromatographié sur gel de silice (éluant : pentane/acétate d'éthyle, 4:1). 26 mg (0,068 mmol) de diastéréoisomère A et 93 mg (0,24 mmol) de diastéréoisomère B sont obtenus.
<u>Rendement</u> : 44%

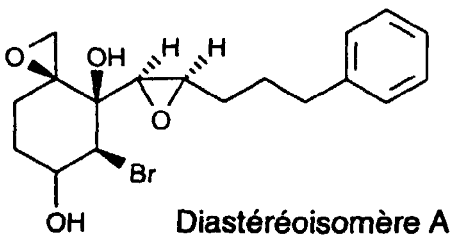

Diastéréoisomère A

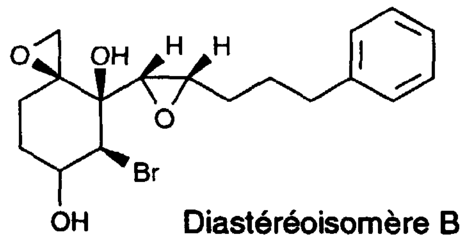

Diastéréoisomère B

Etape D : (3*S**, 4*R**, 5*S**, 6*S**)-5-bromo-6-chloroacétylcarbamoyloxy-4-hydroxy-4-[3-(3-phényl-pentyl)-oxiran-2-yl]-1-oxa-*spiro*[2.5]octane

En procédant selon le mode opératoire décrit à l'étape E de l'exemple 1, à partir de 93 mg (0,24 mmol) de composé obtenu à l'étape C précédente et de 40 ml (0,45 mmol) d'isocyanate de chloroacétyle dans 5 ml de dichlorométhane anhydre, 50 mg (0,1 mmol) de composé attendu sont obtenus après chromatographie sur gel de silice (éluant : heptane/acétate d'éthyle, 1,2:1).
Rendement : 41%

Analyse élémentaire :

| (Formule brute : $C_{21}H_{25}BrClNO_6$ masse moléculaire : 502,79) | | | |
|---|---|---|---|
| | **C** | **H** | **N** |
| **% trouvé** | 50,47 | 5,12 | 2,84 |
| **% calculé** | 50,17 | 5,01 | 2,79 |

**EXEMPLE 14 : 6-Chloroacétylcarbamoyloxy-4-(β-styryl)-1-oxa-*spiro*[2.5]oct-4-ène**

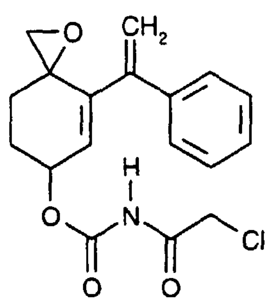

Etape A : 4-(β-styryl)-1-oxa-*spiro*[2.5]oct-4-én-6-one

En procédant selon le mode opératoire décrit à l'étape A de l'exemple 1, à partir de 1,7 ml (13 mmol) de β-bromostyrène, de 15,3 ml de *tert*-butyllithium (1,7 M dans le pentane soit 26 mmol) dans 40 ml de diéthyléther anhydre et de 1g (6,5 mmol) de 6-méthoxy-1-oxa-*spiro*[2.5]oct-5-én-4-one dans 10 ml de toluène anhydre, 0,24 g (0,93 mmol) d'isomère A, 0,42 g (1,63 mmol) d'isomère B et 0,18 g (0,8 mmol) de produit attendu sont obtenus après deux chromatographies sur gel de silice (éluants : pentane/acétate d'éthyle, 5:1 et toluène/acétate d'éthyle, 95:5).
Rendement : 12%

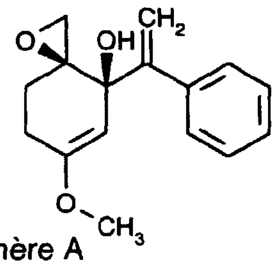
Isomère A

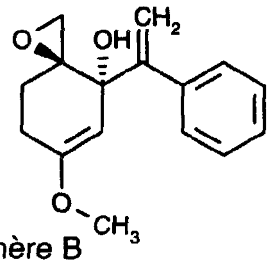
Isomère B

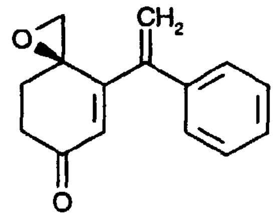
Produit titre

Etape B : 6-hydroxy-4-(β-styryl)-1-oxa-*spiro*[2.5]oct-4-ène

En procédant selon le mode opératoire décrit à l'étape D de l'exemple 1, à partir de 0,16 g (0,71 mmol) de composé obtenu à l'étape A et de 30 mg (0,75 mmol) de borohydrure de sodium dans 22 ml d'un mélange dichlorométhane/méthanol (10:1), 0,14 g (0,61 mmol) de composé attendu sont obtenus après chromatographie sur gel de silice (éluant : heptane/acétate d'éthyle, 1:1).
Rendement : 87%

Etape C : 6-chloroacétylcarbamoyloxy-4-(β-styryl)-1-oxa-*spiro*[2.5]oct-4-ène

En procédant selon le mode opératoire décrit à l'étape E de l'exemple 1, à partir de 0,14 g (0,61 mmol) de composé obtenu à l'étape B et de 0,1 ml (1,14 mmol) d'isocyanate de chloroacétyle dans 10 ml de dichlorométhane anhydre, 0,25 g (0,73 mmol) de composé attendu sont obtenus.

**EXEMPLE 15 : 6-Chloroacétylcarbamoyloxy-4-(2-phényloxiran-2-yl)-1-oxa-*spiro* [2.5]oct-4-ène**

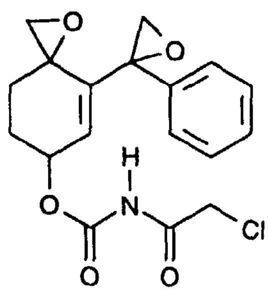

En procédant selon le mode opératoire décrit à l'étape C de l'exemple 13, à partir de 0,25 g (0,73 mmol) de composé brut obtenu dans l'exemple 14 dans 25 ml de diméthyldioxirane, 90 mg (0,25 mmol) de composé attendu sont obtenus après chromatographie sur gel de silice (éluant : heptane/acétate d'éthyle, 2:1).
Rendement : 39%

Analyse élémentaire :

| (Formule brute : $C_{18}H_{18}ClNO_5$ masse moléculaire : 363,80) | | | |
|---|---|---|---|
| | **C** | **H** | **N** |
| **% trouvé** | 59,21 | 4,98 | 3,84 |
| **% calculé** | 59,43 | 4,99 | 3,85 |

**EXEMPLE 16 : (1*S**, 2*S**, 5*S**, 6*S**)-5-Chloroacétylcarbamoyloxy-1-isopropényl-2-(2-*spiro*-oxiranyl)-7-oxabicyclo[4.1.0]heptane**

(±) 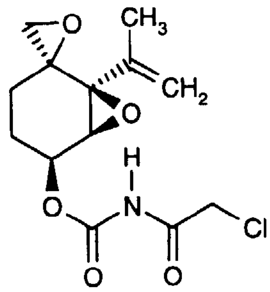

Etape A : 5-hydroxy-1-isopropényl-2-(2-*spiro*-oxiranyl)-7-oxabicydo[4.1.0]heptane

En procédant selon le mode opératoire décrit à l'étape D de l'exemple 1, à partir de 2 g (7,65 mmol) de 5-bromo-4-hydroxy-4-isopropényl-1-oxa-*spiro*[2.5]octan-6-one (isomère A) obtenu comme dans l'étape C de l'exemple 3 et de 0,29 g (7,66 mmol) de borohydrure de sodium dans 220 ml d'un mélange dichlorométhane/méthanol (10:1), 0,69 g (3,79 mmol) d'isomère 1 et 1,2 g (6,59 mmol) d'isomère 2 attendus sont obtenus après chromatographie sur gel de silice (éluant : pentane/acétate d'éthyle, 4:1).

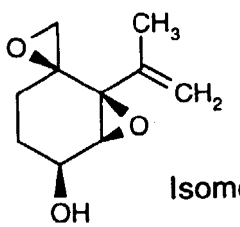

Isomère 1

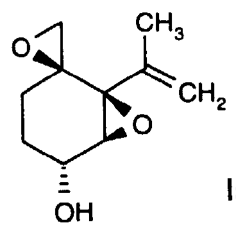

Isomère 2

Etape B : ( 1*S**, 2*S**, 5*S**, 6*S**)-5-chloroacétylcarbamoyloxy-1-isopropényl-2-(2-*spiro*-oxiranyl)-7-oxabicyclo[4.1.0]heptane

En procédant selon le mode opératoire décrit à l'étape E de l'exemple 1, à partir de 0,53 g (2,91 mmol) d'isomère 1 obtenu à l'étape A précédente et de 0,46 ml (5,41 mmol) d'isocyanate de chloroacétyle dans 30 ml de dichlorométhane anhydre, 0,77g (2,54 mmol) de composé attendu sont obtenus après chromatographie sur gel de silice (éluant : heptane/acétate d'éthyle, 2:1).

Rendement : 87%

Analyse élémentaire :

| (Formule brute : $C_{13}H_{16}ClNO_5$ masse moléculaire : 301,73) | | | | |
|---|---|---|---|---|
| | C | H | N | Cl |
| **% trouvé** | 51,72 | 5,58 | 4,61 | 11,90 |
| **% calculé** | 51,75 | 5,35 | 4,64 | 11,75 |

Cette synthèse a été réalisée dans les mêmes conditions opératoires avec l'isomère 2 obtenu à l'étape A, afin d'obtenir le composé de l'exemple 29.

**EXEMPLE 17 : (1*R**, 2*S**, 5*S**, 6*S**)-5-Chloroacétylcarbamoyloxy-1-[(2*S** et 2*R**)-(2-méthyloxiran-2-yl)]-2-(2-*spiro*-oxiranyl)-7-oxabicyclo[4.1.0]heptane**

(±) 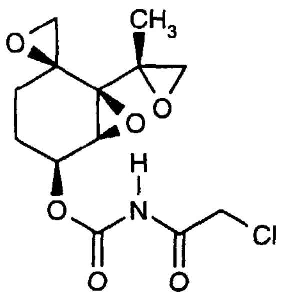

Diastéréoisomère A

(±) 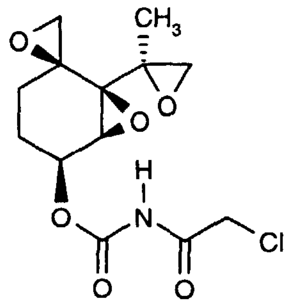

Diastéréoisomère B

En procédant selon le mode opératoire décrit dans l'exemple 2, à partir de 0,61 g (2,01 mmol) de composé obtenu dans l'exemple 16 et de 1,96 g (16,1 mmol) d'acide 3-chloroperbenzoïque à 72% dans 50 ml de dichlorométhane, on obtient 0,13g (0,41 mmol) de diastéréoisomère A et 0,08 g (0,25 mmol) de diastéréoisomère B après chromatographie sur gel de silice (éluant : heptane/acétate d'éthyle, 1:1) et HPLC sur RP18 (éluant : acétonitrileleau, 20:80).
Rendement : 32%

Analyse élémentaire des diastéréoisomères A et B :

(Formule brute : $C_{13}H_{16}ClNO_6$ masse moléculaire : 317,73)

**Diastéréoisomère A**

| | C | H | N | Cl |
|---|---|---|---|---|
| **% trouvé** | 49,10 | 5,04 | 4,36 | 11,21 |
| **% calculé** | 49,14 | 5,08 | 4,41 | 11,16 |

**Diastéréoisomère B**

| | C | H | N | Cl |
|---|---|---|---|---|
| **% trouvé** | 49,18 | 4,98 | 4,48 | 11,52 |
| **% calculé** | 49,14 | 5,08 | 4,41 | 11,16 |

Cette synthèse a été réalisée dans les mêmes conditions opératoires à partir de l'isomère 2 obtenu dans l'exemple 16, afin d'obtenir les deux diastéréoisomères décrits à l'exemple 30.

**EXEMPLE 18 : (1*S**, 2*S**, 5*R** 6*S**)5-Chloroacétylcarbamoyloxy-2-(2-*spiro*-oxi-ranyl)-1-5-phényl-pentyl)-7-oxabicyclo[4.1.0]heptane**

(±) 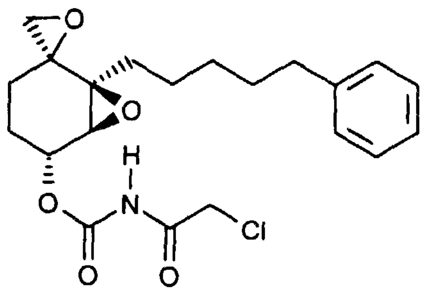

Etape A : 5-hydroxy-2-(2-*spiro*-oxiranyl)-1-(5-phénylpentyl)-7-oxabicyclo[4.1.0] heptane

En procédant selon le mode opératoire décrit à l'étape D de l'exemple 1, à partir de 4,2 g (11,5 mmol) de l'isomère C obtenu à l'étape A de l'exemple 10 et de 0,22 g (5,8 mmol) de borohydrure de sodium dans 440 ml d'un mélange dichlorométhane/méthanol (10:1), 1,6 g (5,45 mmol) de composé attendu sont obtenus.

Etape B : 5-chloroacétylcarbamoyloxy-2-(2-*spiro*-oxiranyl)-1-(5-phénylpentyl)-7-oxabicyclo[4.1.0]heptane

En procédant selon le mode opératoire décrit à l'étape E de l'exemple 1, à partir de 1,6 g (5,45 mmol) du composé obtenu à l'étape A précédente et de 0,85 ml (10,14 mmol) d'isocyanate de chloroacétyle, 0,16 g (0,39 mmol) du composé attendu, après deux chromatographies sur gel de silice (éluant: heptane/acétate d'éthyle, 2:1), sont obtenus.

Analyse élémentaire :

| (Formule brute : $C_{21}H_{26}ClNO_5$ masse moléculaire : 407,90) | | | | |
|---|---|---|---|---|
| | **C** | **H** | **N** | **Cl** |
| **% trouvé** | 61,81 | 6,48 | 3,34 | 8,64 |
| **% calculé** | 61,84 | 6,42 | 3,43 | 8,69 |

**EXEMPLE 19 : (1*S**, 2*R**, 5*R**, 6*S**)-2,5.Dichloroacétylcarbamoyloxy-2-méthyl-1-(5-phénylpentyl)-7-oxabicyclo [4.1.0]heptane**

(±) 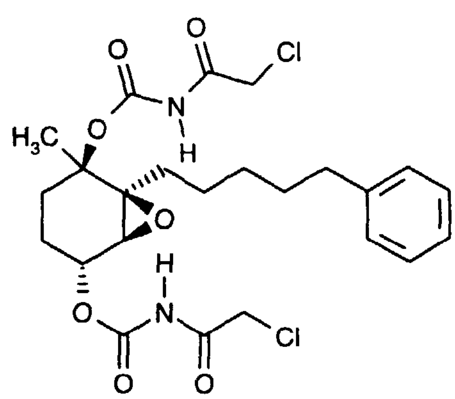

Etape A : 2,5-dihydroxy-2-méthyl-1-(5-phénylpentyl)-7-oxabicyclo[4.1.0]heptane

En procédant selon le mode opératoire décrit à l'étape D de l'exemple 1, à partir de 4,2 g (11,5 mmol) de l'isomère E obtenu à l'étape A de l'exemple 10 et de 0,22 g (5,8 mmol) de borohydrure de sodium dans 440 ml d'un mélange

dichlorométhane/méthanol (10:1), 1,6 g (5,45 mmol) de composé attendu sont obtenus.

Etape B : (1*S**, 2*R**, 5*R**, 6*S**)-2,5-dichloroacétylcarbamoyloxy-2-méthyl-1-(5-phénylpentyl)-7-oxabicyclo[4.1.0]heptane

En procédant selon le mode opératoire décrit à l'étape E de l'exemple 1, à partir de 1,6 g (5,45 mmol) du composé obtenu à l'étape A précédente et de 0,85 ml (10,14 mmol) d'isocyanate de chloroacétyle, 0,17 g (0,32 mmol) du composé attendu, après deux chromatographies sur gel de silice (éluant : heptane/acétate d'éthyle, 2:1), sont obtenus.

Analyse élémentaire :

| (Formule brute : $C_{24}H_{30}Cl_2N_2O_7$ masse moléculaire : 529,42) | | | | |
|---|---|---|---|---|
| | C | H | N | Cl |
| **% trouvé** | 54,87 | 5,90 | 4,95 | 13,00 |
| **% calculé** | 54,45 | 5,71 | 5,29 | 13,39 |

**EXEMPLE 20 : 6-Chloroacétylcarbamoyloxy-4-(4-méthylpentyl)-1-oxa-*spiro*[2.5] oct-4-ène**

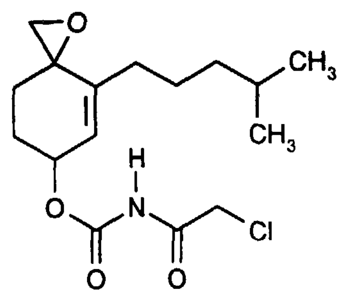

Etape A : 4-(4-méthylpentyn-1-yl)-1-oxa-*spiro*[2.5]oct-4-én-6-one

A une solution de 2 g (24,34 mmol) de 4-méthylpentyne dans 80 ml de diéthyléther anhydre refroidie à -78°C sont ajoutés goutte-à-goutte et sous atmosphère d'azote 16 ml (25,5 mmol) de n-butyllithium (1,6 M dans l'hexane). L'ensemble est laissé sous agitation à -78°C pendant 15 minutes puis à 0°C pendant 50 minutes. A cette solution est alors additionnée goutte-à-goutte une solution de 6-méthoxy-1-oxa-*spiro*[2.5]oct-5-én-4-one (2,5 g ; 16,21 mmol) dans 30 ml de toluène anhydre refroidie à -78°C. Après 1 heure d'agitation à -78°C, le mélange réactionnel est versé dans une solution aqueuse à 10% de chlorure d'ammonium. Le traitement habituel de la phase organique fournit après chromatographie sur gel de silice (éluant : pentane/acétate d'éthyle, 4:1) 1g (4,25 mmol) d'isomère 1 et 1g (4,25 mmol) d'un mélange d'isomère 2 et d'isomère 3 de réarrangement que l'on utilise sans purification pour l'étape suivante.

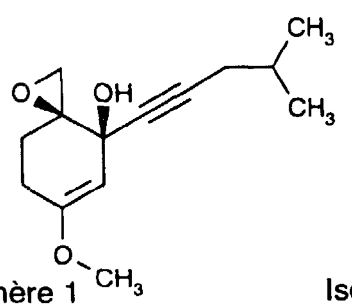

Isomère 1

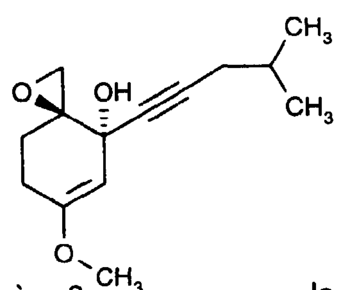

Isomère 2

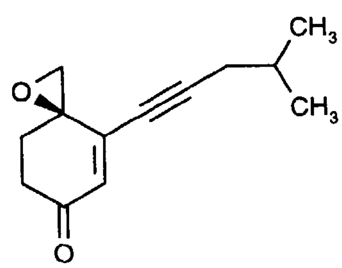

Isomère 3

Etape B : 4-(4-méthylpentyl)-1-oxa-*spiro*[2,5]oct-4-én-6-one

L'hydrogénation d'une solution de 1 g (4,25 mmol) du mélange d'isomère 2 et d'isomère 3 obtenu à l'étape A dans 140 ml de benzène en présence de 0,28 g de catalyseur de Lindlar pendant 90 minutes conduit après filtration et évaporation à 0,4 g (1,92 mmol) de composé attendu après chromatographie sur gel de silice (éluant : pentane/acétate d'éthyle ; 7:1).
Rendement : 45%

Etape C : 6-hydroxy-4-(4-méthylpentyl)-1-oxa-*spiro*[2.5]oct-4-ène

En procédant selon le mode opératoire décrit à l'étape D de l'exemple 1, à partir de 0,36 g (1,75 mmol) de composé obtenu à l'étape précédente et de 68 mg (1,8 mmol) de borohydrure de sodium dans 55 ml d'un mélange dichlorométhane/méthanol (10:1), 0,17 g (0,82 mmol) de composé attendu sont obtenus après chromatographie sur gel de silice (éluant : heptane/acétate d'éthyle, 3:1).
Rendement : 76%

Etape D : 6-chloroacétylcarbamoyloxy-4-(4-méthylpentyl)-1-oxa-*spiro*[2.5]oct-4-ène

En procédant selon le mode opératoire décrit à l'étape E de l'exemple 1, à partir de 0,17 g (0,82 mmol) de composé obtenu à l'étape précédente et de 0,14 ml (1,55 mmol) d'isocyanate de chloroacétyle dans 10 ml de dichlorométhane anhydre, 0,3 g (0,91 mmol) de composé attendu sont obtenus.

**EXEMPLE 21 : 5-Chloroacétylcarbamoyloxy-2-(2-*spiro*-oxiranyl)-1-4-méthylpentyl)-7-oxabicyclo[4.1.0]heptane**

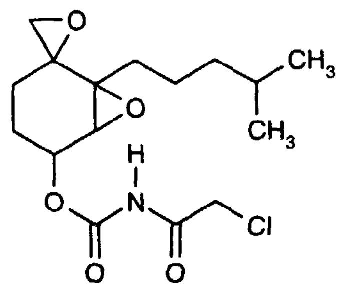

En procédant selon le mode opératoire décrit à l'étape C de l'exemple 13, à partir de 0,3 g (0,91 mmol) de composé obtenu à l'exemple 20 dans 20 ml de diméthyldioxirane, 22 mg (0,063 mmol) de composé attendu sont obtenus après deux chromatographies sur gel de silice (éluant : toluène/acétate d'éthyle, 9:1).
Rendement : 7%

Analyse élémentaire :

| (Formule brute : $C_{16}H_{24}ClNO_5$ masse moléculaire : 345,82) | | | |
|---|---|---|---|
| | C | H | N |
| **% trouvé** | 55,83 | 7,17 | 3,78 |
| **% calculé** | 55,57 | 7,00 | 4,05 |

**<u>EXEMPLE 22</u> : (3*S**, 4*R**, 5*R**, 6*R**)-6-Chloroacétylcarbamoyloxy-4-isopropényl-1-oxa-*spiro*[2.5]hexahydrobenzo[1.3]dioxol-2-one**

(±)

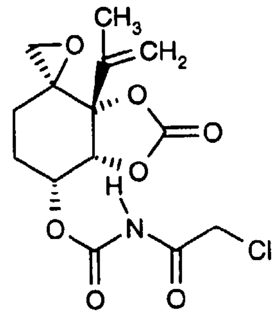

<u>Etape A</u> : 4,5-dihydroxy-4-isopropényl-1-oxa-*spiro*[2.5]octan-6-one

En procédant selon le mode opératoire décrit à l'étape C de l'exemple 13, à partir de 1,8 g (9,2 mmol) d'isomère B obtenu à l'étape A de l'exemple 3 dans 100 ml de diméthyldioxirane, 1,7 g (8,6 mmol) de composé attendu sont obtenus après chromatographie sur gel de silice (éluant : pentane/acétate d'éthyle, 4:1).
<u>Rendement</u> : 93%

<u>Etape B</u> : 4,5,6-trihydroxy-4-isopropényl-1-oxa-*spiro*[2.5]octane

En procédant selon le mode opératoire décrit à l'étape D de l'exemple 1, à partir de 0,59 g (2,98 mmol) de composé obtenu à l'étape précédente et de 0,11g (2,98 mmol) de borohydrure de sodium dans 66 ml d'un mélange dichlorométhane/méthanol (10:1), 0,48 g (2,4 mmol) de composé brut attendu sont obtenus.
<u>Rendement</u> : 80%

<u>Etape C</u> : 5,6-dichloroacétylcarbamoyloxy-4-hydroxy-4-isopropényl-1-oxa-*spiro* [2.5]octane

En procédant selon le mode opératoire décrit à l'étape E de l'exemple 1, à partir de 0,48 g (2,4 mmol) de composé brut obtenu à l'étape précédente et de 0,76 ml (8,89 mmol) d'isocyanate de chloroacétyle dans 40 ml de dichlorométhane anhydre, 1,3 g (2,96 mmol) de composé brut attendu sont obtenus.

<u>Etape D</u> : (3*S**, 4*R**, 5*R**, 6*R**)-6-chloroacétylcarbamoyloxy-4-isopropényl- 1-oxa-*spiro*[2.5]hexa-hydro-benzo[1.3] dioxol-2-one

A une solution de 1,3 g (2,96 mmol) de composé brut obtenu à l'étape précédente dans 100 ml de dichlorométhane sont ajoutés 40 g de gel de silice. Après 2 jours d'agitation à température ambiante, le mélange réactionnel est filtré puis évaporé. Après chromatographie sur gel de silice (éluant : heptane/acétate d'éthyle, 2:1), 0,18 g (0,52 mmol) de composé attendu sont obtenus.
<u>Rendement</u> : 18%

<u>Analyse élémentaire</u> :

| (Formule brute : $C_{14}H_{16}ClNO_7$ masse moléculaire : 345,74) | | | | |
|---|---|---|---|---|
| | **C** | **H** | **N** | **Cl** |
| **% trouvé** | 49,08 | 4,99 | 3,82 | 10,06 |
| **% calculé** | 48,64 | 4,66 | 4,05 | 10,25 |

**EXEMPLE 23 : (3*S**, 4*S**, 5*R**, 6*R**)-6-Chloroacétylcarbamoyloxy-4-(2-méthyloxiran-2-yl)-1-oxa-*spiro*[2.8]benzo [1.3]dioxol-2-one**

(±)

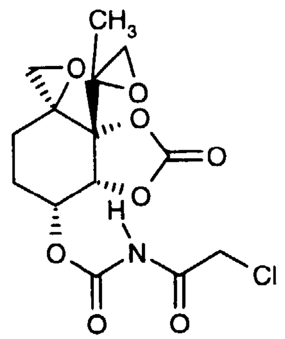

En procédant selon le mode opératoire décrit à l'exemple 2, à partir de 0,78 g (1,78 mmol) de composé brut obtenu à l'étape D de l'exemple 22 et de 0,84 g (3,42 mmol) d'acide 3-chloroperbenzoïque à 72%, 0,1g (0,28 mmol) de composé attendu sont obtenu après deux chromatographies sur gel de silice (éluant: hexane/acétate d'éthyle, 1:1) et HPLC sur RP18 (éluant : acétonitrile/eau, 30:70).
Rendement : 15%

Analyse élémentaire :

| (Formule brute : $C_{14}H_{16}ClNO_8$ masse moléculaire : 361,74) | | | |
|---|---|---|---|
| | **C** | **H** | **N** |
| **% trouvé** | 46,03 | 4,17 | 4,07 |
| **% calculé** | 46,49 | 4,46 | 3,87 |

Les trois composés suivants ont été obtenus respectivement de manière analogue aux composés des exemples 12, 27 et 28.

**EXEMPLE 24 (1): (3*S**, 4*R**, 6*S**)-4,6-Dichloroacétylcarbamoyloxy-4-hexyl-1-oxa-*spiro*[2.5]octane**

(±)

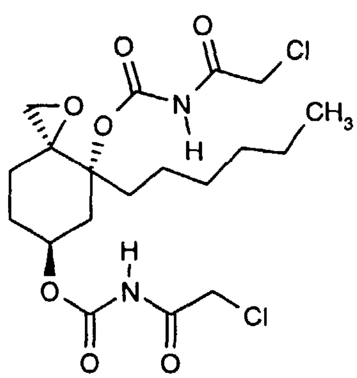

**EXEMPLE 24 (2) : (3*S**, 4*R**, 6*R**)-4,6-Dichloroacétylcarbamoyloxy-4-hexyl-1-oxa-*spiro*[2.5]octane**

(±)

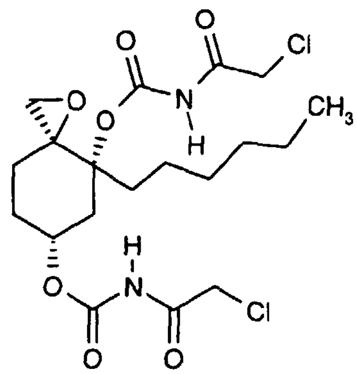

**EXEMPLE 24 (3) : (3*S**, 4*S**, 6*S**)-4,6-Dichloroacétylcarbamoyloxy-4-hexyl-1-oxa-*spiro*[2.5]octane**

(±)

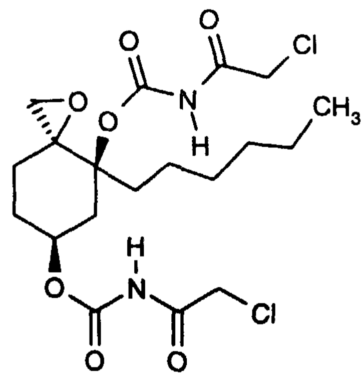

Analyse élémentaire :

| (Formule brute : $C_{19}H_{28}Cl_2N_2O_7$ masse moléculaire : 467,35) | | | |
|---|---|---|---|
| | **C** | **H** | **N** |
| **% trouvé** | 49,08 | 6,26 | 5,60 |
| **% calculé** | 48,83 | 6,04 | 5,99 |

De façon identique, les composés 25(1) à 25(3) ont également été obtenus.

**<u>EXEMPLE 25</u> (1) : (3*S**, 4*S**, 6*S**)-4,6-Dichloroacétylcarbamoyloxy-4-isopropyl-1-oxa-*spiro*[2.5]octane**

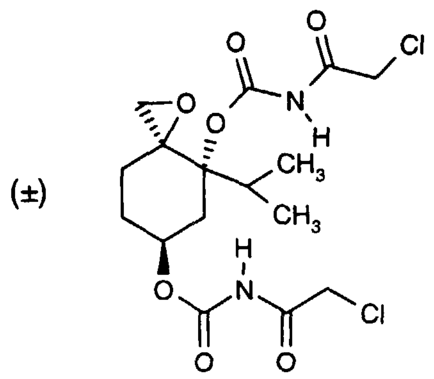

Analyse élémentaire :

| (Formule brute : $C_{16}H_{22}Cl_2N_2O_7$ masse moléculaire : 425,27) | | | |
|---|---|---|---|
| | **C** | **H** | **N** |
| **% trouvé** | 45,92 | 5,44 | 6,06 |
| **% calculé** | 45,19 | 5,21 | 6,59 |

**<u>EXEMPLE 25</u> (2): (3*S**, 4*S**, 6*R**)-4,6-Dichloroacétylcarbamoyloxy-4-isopropyl-1-oxa-spiro[2.5]octane**

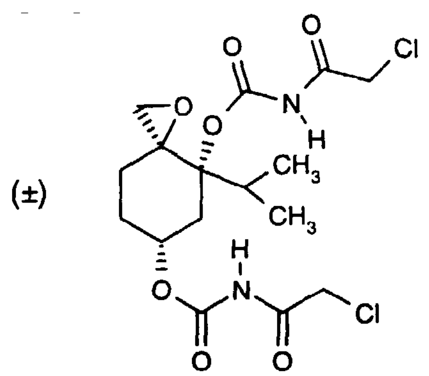

Analyse élémentaire :

| (Formule brute : $C_{16}H_{22}Cl_2N_2O_7$ masse moléculaire : 425,27) | | | |
|---|---|---|---|
| | **C** | **H** | **N** |
| **% trouvé** | 45,97 | 5,39 | 6,27 |
| **% calculé** | 45,19 | 5,21 | 6,59 |

**<u>EXEMPLE 25</u> (3) : (3*S**, 4*R**, 6*S**)-4,6-Dichloroacétylcarbamoyloxy-4-isopropyl-1-oxa-*spiro*[2.5]octane**

(±) 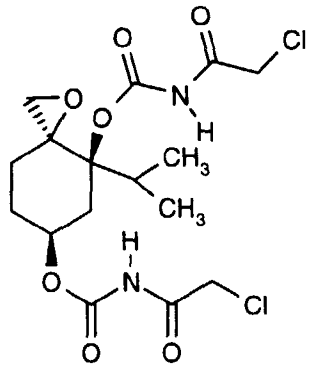

**<u>EXEMPLE 26</u> : (3*S**, 4*S**, 6*S**)-6-Chloroacétylcarbamoyloxy-4-hydroxy-4-(5-phénylpentyl)-1-oxa-*spiro*[2.5]octane**

(±) 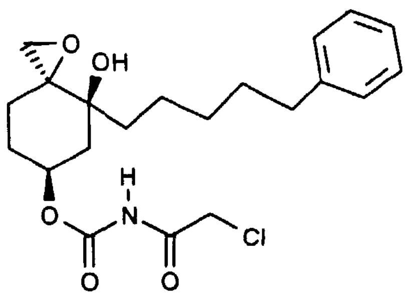

En procédant comme décrit pour l'exemple 11 mais à partir de l'isomère B obtenu à l'étape B de l'exemple 10, le composé titre est obtenu.

<u>Analyse élémentaire</u> :

| (Formule brute : $C_{21}H_{28}ClNO_5$ masse moléculaire : 409,91) | | | |
|---|---|---|---|
| | **C** | **H** | **N** |
| **% trouvé** | 61,24 | 7,07 | 3,42 |
| **% calculé** | 61,53 | 6,89 | 3,42 |

**<u>EXEMPLE 27</u> : (3*S**, 4*R**, 6*R**)-4,6-Dichloroacétylcarbamoyloxy-4-(5-phénylpentyl)-1-oxa-*spiro*[2.5]octane**

(±) 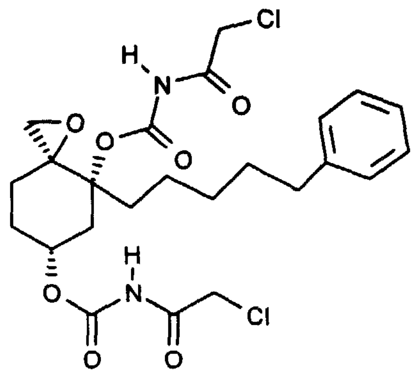

En procédant comme décrit pour l'exemple 12 à partir de l'isomère A obtenu à l'étape B de l'exemple 10, le composé titre est obtenu.

<u>Analyse élémentaire</u> :

| (Formule brute : $C_{24}H_{30}Cl_2N_2O_7$ masse moléculaire : 529,42) | | | | |
|---|---|---|---|---|
| | **C** | **H** | **N** | **Cl** |
| **% trouvé** | 54,18 | 5,72 | 5,41 | 13,59 |
| **% calculé** | 54,45 | 5,71 | 5,29 | 13,39 |

**<u>EXEMPLE 28</u> : (3*S**, 4*S**, 6*S**)-4,6-Dichloroacétylcarbamoyloxy-4-(5-phénylpentyl)-1-oxa-*spiro*[2.5]octane**

(±) 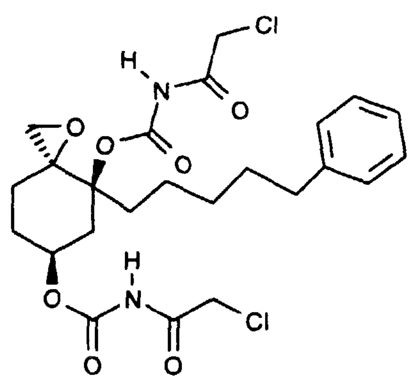

En procédant comme décrit pour l'exemple 12 à partir de l'isomère B obtenu à l'étape B de l'exemple 10, le composé titre est obtenu.

<u>Analyse élémentaire</u> :

| (Formule brute : $C_{24}H_{30}Cl_2N_2O_7$ masse moléculaire : 529,42) | | | |
|---|---|---|---|
| | **C** | **H** | **N** |
| **% trouvé** | 54,27 | 6,04 | 4,77 |
| **% calculé** | 54,45 | 5,71 | 5,29 |

**<u>EXEMPLE 29</u> : (1*S**, 2*S**, 5*R**, 6*S**)-5-Chloroacétylcarbamoyloxy-1-isopropényl-2-(2-*spiro*-oxiranyl)-7-oxabicyclo[4.1.0]heptane**

(±) 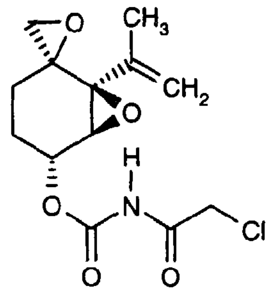

En procédant comme décrit dans l'exemple 16 à partir de l'isomère 2, le composé titre est obtenu.

<u>Analyse élémentaire</u> :

| (Formule brute : $C_{13}H_{16}ClNO_5$ masse moléculaire : 301,73) | | | | |
|---|---|---|---|---|
| | **C** | **H** | **N** | **Cl** |
| **% trouvé** | 51,72 | 5,58 | 4,61 | 11,90 |
| **% calculé** | 51,75 | 5,35 | 4,64 | 11,75 |

**<u>EXEMPLE 30</u> : (1*R**, 2*S**, 5*R**, 6*S**)-5-Chloroacétylcarbamoyloxy-1-[(2*S** et 2*R**)-(2-méthyloxiran-2-yl)]-2-(2-*spiro*-oxiranyl)-7-oxabicyclo[4.1.0]heptane**

(±) 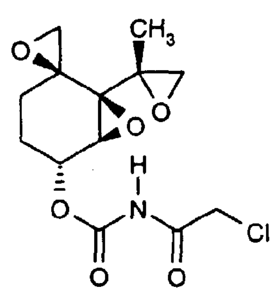

**Diastéréoisomère A**

(±) 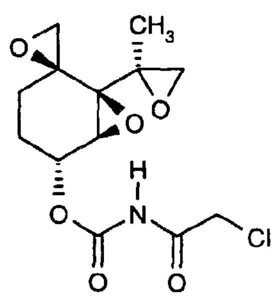

**Diastéréoisomère B**

En procédant comme décrit à l'exemple 17 mais à partir de l'isomère 2 de l'exemple 16, les deux diastéréoisomères attendus sont obtenus.

<u>Spectre de masse</u> : (masse moléculaire : 317) DCI ($NH_3$) : M + $NH_4^+$ = 335

**EXEMPLE 31 : (3*S**, 4*S**)-4-Chloroacétylcarbamoyloxy-4-(5-phénylpentyl)-1-oxa-*spiro*[2.5]octan-6-one**

(±)

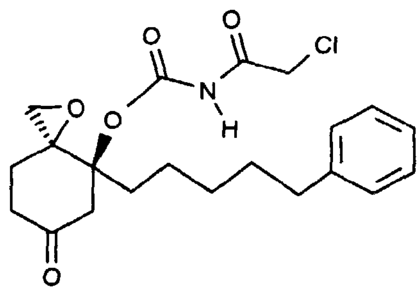

Etape A : 4-hydroxy-4-(5-phénylpentyl)-1-oxa-*spiro*[2.5]octan-6-one

En procédant selon le mode opératoire décrit à l'étape A de l'exemple 5, à partir de 5,2 g (14,16 mmol) du mélange d'isomères C et D obtenu à l'étape A de l'exemple 10 sous leur forme cétonique après action d'acide paratoluènesulfonique, et de 5,8 ml (21,84 mmol) d'hydrure de tributylétain dans 75 ml de toluène anhydre, 3,18 g (11,03 mmol) du composé attendu sont obtenus après chromatographie sur gel de silice (éluant : pentane / acétate d'éthyle, 2:1). Rendement : 78%

Etape B : (3*S**, 4*S**)-4-Chloroacétylcarbamoyloxy-4-(5-phénylpentyl)-1-oxa-*spiro* [2.5)octan-6-one

En procédant selon le mode opératoire décrit à l'étape E de l'exemple 1, à partir de 3,13 g (10,85 mmol) du composé obtenu à l'étape précédente, et de 1,82 g (20,19 mmol) d'isocyanate de chloroacétyle dans 100 ml de dichlorométhane anhydre puis purification par chromatographie phase liquide sur silice RP 18 (éluant : acétonitrile/eau, 575:425), 3,68 g (9,02 mmol) de composé attendu sont obtenus. Rendement : 83%

Analyse élémentaire :

| (Formule brute : $C_{21}H_{26}ClNO_5$ masse moléculaire : 407,90) | | | |
|---|---|---|---|
| | **C** | **H** | **N** |
| **% trouvé** | 61,87 | 6,47 | 3,12 |
| **% calculé** | 61,84 | 6,42 | 3,43 |

**EXEMPLE 32 : (3*S**, 4*R**)-4-Chloroacétylcarbamoyloxy-4-(5-phénylpentyl)-1-oxa-*spiro*[2.5]octan-6-one**

(±)

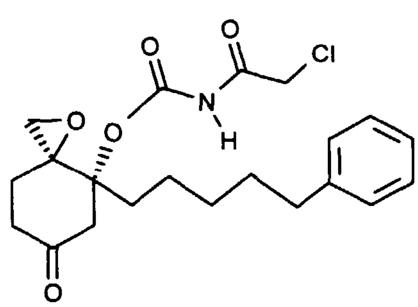

En procédant comme décrit pour l'exemple 31 mais en utilisant le mélange d'isomères A et B obtenu à l'étape A de l'exemple 10, sous leur forme cétonique après action d'acide paratoluènesulfonique, le composé titre est obtenu.

Analyse élémentaire :

| (Formule brute : $C_{21}H_{26}ClNO_5$ masse moléculaire : 407,90) | | | |
|---|---|---|---|
| | **C** | **H** | **N** |
| **% trouvé** | 61,81 | 6,34 | 3,30 |
| **% calculé** | 61,84 | 6,42 | 3,43 |

**EXEMPLE 33 : 4-(5-phénylpentyl)-1-oxa-*spiro*[2.5]oct-4-én-6-one**

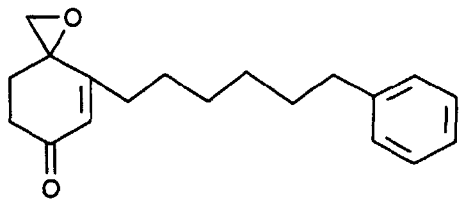

Le traitement par le sulfate de magnésium, en solution dans l'acétate d'éthyle, du composé obtenu dans l'exemple 32 conduit au composé du titre.

Analyse élémentaire :

| (Formule brute : $C_{18}H_{22}O_2$ masse moléculaire : 270,37) | | |
|---|---|---|
| | **C** | **H** |
| **% trouvé** | 79,37 | 8,17 |
| **% calculé** | 79,96 | 8,20 |

**EXEMPLE 34 : (1*S**, 2*S**, 4*S**)-1-Chloroacétylcarbamoyloxyméthyl-4-chloroacétylcarbamoyloxy-2-(5-phényl-pentyl)cyclohexane**

(±)

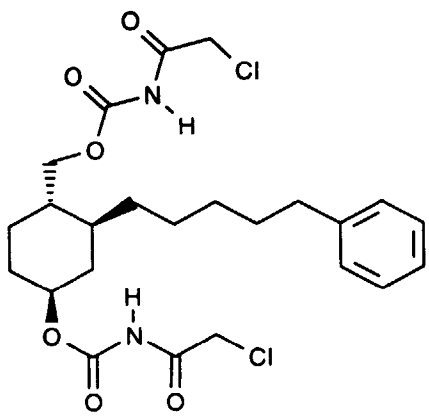

Etape A : 4-hydroxy-6-méthoxy-4-(5-phénylpent-1-ynyl)-1-oxa-*spiro*[2.5]oct-5-ène

En procédant selon le mode opératoire décrit à l'étape A de l'exemple 10, à partir de 25 ml (0,157 moles) de 5-phénylpentyne dans 600 ml de diéthyléther en présence de 100 ml (0,150 moles) de n-butyllithium, et de 15 g (0,097 moles) de 6-méthoxy-1-oxa-*spiro*[2.5]oct-5-én-4-one dans 80 ml de toluène anhydre, 26,94 g du composé attendu sont obtenus.
Rendement : 93%

Etape B : 4-hydroxyméthyl-3-(5-phénylpentyl)cyclohex-2-énone

L'hydrogénation d'une solution de 24,9 g (83,45 mmol) du composé obtenu à l'étape précédente dans 1 l de benzène en présence de 7,5 g de catalyseur de Pearlmann pendant 75 minutes, conduit, après filtration et évaporation du solvant, à 9,67 g (35,50 mmol) du produit attendu et 2,83 g (10,46 mmol) de 4-(5-phénylpentyl)-1-oxa-*spiro*[2.5] oct-4-én-5-one, après chromatographie sur gel de silice (éluant : heptane/acétate d'éthyle, 4:1).
Rendement : 55%

Etape C : 4-hydroxyméthyl-3-(5-phénylpentyl)cyclohex-2-énol (A et B) et 4-hydroxyméthyl)-3-(5-phénylpentyl)cyclohexanol (C)

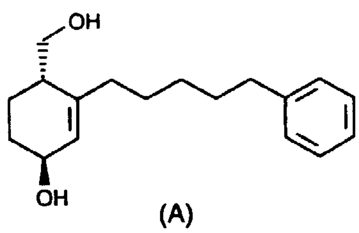
(A)

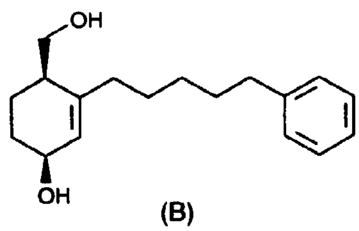
(B)

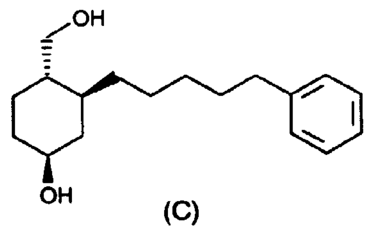
(C)

En procédant selon le mode opératoire décrit à l'étape D de l'exemple 1, à partir de 11,6 g (42,58 mmol) du composé obtenu à l'étape précédente et de 1,6 g (42,28 mmol) de borohydrure de sodium dans 100 ml d'un mélange dichlorométhane/méthanol (17:3), les composés A, B et C obtenus en mélange sont séparés par chromatographie sur colonne de silice (éluant : toluène/acétate d'éthyle, 15:1) pour foumir :

A : 3,57 g (13,01 mmol)
B : 4,5 g (16,28 mmol)
C : 0,88 g (3,18 mmol)

Rendement global : 76%

Etape D : (1*S**, 2*S**, 4*S**)-1-Chloroacétylcarbamoyloxyméthyl-4-chloroacétylcarbamoyloxy-2-(5-phénylpentyl)cyclohexane

En procédant selon le mode opératoire décrit à l'étape E de l'exemple 1, à partir de 0,7 g (2,5 mmol) du composé C obtenu à l'étape précédente dans 25 ml de dichlorométhane et de 0,76 ml (5,96 mmol) d'isocyanate de chloroacétyle, traitement habituel de la phase organique, puis précipitation dans un mélange acétate d'éthyle/pentane/éther diéthylique, 0,98 g (1,9 mmol) du composé attendu sont obtenus.
Rendement : 75%

Analyse élémentaire :

| (Formule brute $C_{24}H_{32}Cl_2N_2O_6$ masse moléculaire : 515,44) | | | | |
|---|---|---|---|---|
| | **C** | **H** | **N** | **Cl** |
| **% trouvé** | 55,31 | 6,28 | 5,33 | 13,69 |
| **% calculé** | 55,93 | 6,26 | 5,43 | 13,76 |

**<u>EXEMPLE 35</u> : (1*S**, 4*S**)-1-Chloroacétylcarbamoyloxyméthyl-4-chloroacétylcarbamoyloxy-2-(5-phénylpentyl) cyclohex-2-ène**

(±)

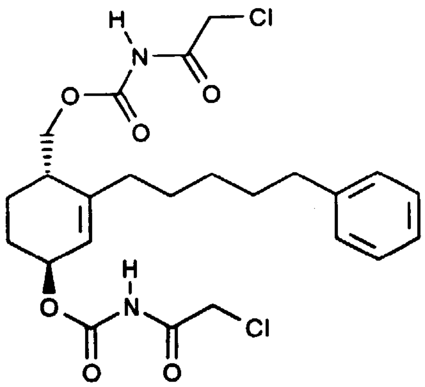

En procédant selon le mode opératoire décrit à l'Exemple précédent, à partir de 1 g (3,64 mmol) du composé A obtenu à l'étape C de l'exemple précédent, dans 36 ml de dichlorométhane et en présence de 1,11 ml (13,56 mmol) d'isocyanate de chloroacétyle, 1 g (1,95 mmol) du composé attendu est obtenu.
<u>Rendement</u> : 53%

<u>Analyse élémentaire</u> :

| (Formule brute $C_{24}H_{30}Cl_2N_2O_6$ masse moléculaire : 513,42) | | | | |
|---|---|---|---|---|
| | **C** | **H** | **N** | **Cl** |
| **% trouvé** | 56,11 | 6,04 | 5,42 | 14,14 |
| **% calculé** | 56,15 | 5,89 | 5,46 | 13,81 |

**<u>EXEMPLE 36</u> : (1*R**, 4*S**)-1-Chloroacétylcarbamoyloxyméthyl-4-chloroacétylcarbamoyloxy-2-(5-phénylpentyl) cyclohex-2-ène**

(±)

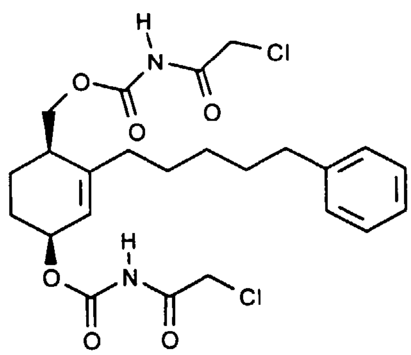

En procédant selon le mode opératoire décrit à l'Exemple précédent, à partir de 1,62 g (5,90 mmol) du composé B obtenu à l'étape C de l'exemple précédent, dans 32 ml de dichlorométhane et en présence de 1,87 ml (21,96 mmol) d'isocyanate de chloroacétyle, 2,4 g (4,67 mmol) du composé attendu est obtenu.
<u>Rendement</u> : 80%

Analyse élémentaire :

| (Formule brute $C_{24}H_{30}Cl_2N_2O_6$ masse moléculaire : 513,42) | | | | |
|---|---|---|---|---|
| | **C** | **H** | **N** | **Cl** |
| **% trouvé** | 56,27 | 5,89 | 5,45 | 13,99 |
| **% calculé** | 56,15 | 5,89 | 5,46 | 13,81 |

**EXEMPLE 37 : (2*S**, 5*S**)-2-Chloroacétylcarbamoyloxyméthyl-5-chloroacétylcarbamoyloxy-1-(5-phénylpentyl)-7-oxabicyclo[4.1.0]heptane**

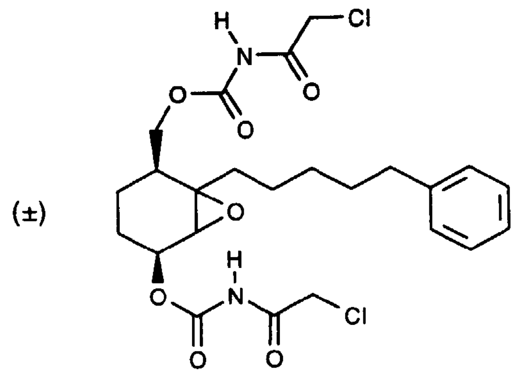

En procédant selon le mode opératoire décrit à l'étape C de l'exemple 13, à partir de 1 g (1,95 mmol) du composé de l'exemple 36 et de 200 ml de diméthyldioxirane, 0,611 g (11,45 mmol) de composé attendu sont obtenus après chromatographie HPLC en phase inverse (silice greffée C18) (élaunt : acétonitrile/eau, 60:40).
Rendement : 59%

Analyse élémentaire :

| (Formule brute $C_{24}H_{30}Cl_2N_2O_7$ masse moléculaire : 529,42) | | | | |
|---|---|---|---|---|
| | **C** | **H** | **N** | **Cl** |
| **% trouvé** | 54,57 | 5,80 | 5,24 | 13,39 |
| **% calculé** | 54,45 | 5,71 | 5,29 | 13,39 |

**EXEMPLE 38 : (3S*, 4S*, 5R*, 6S*)-5-Bromo-6-chloroacétylcarbamoyloxy-4-hydroxy-4-(2-méthyl-2-oxiranyl)-1-oxa-*spiro*[2.5]octane**

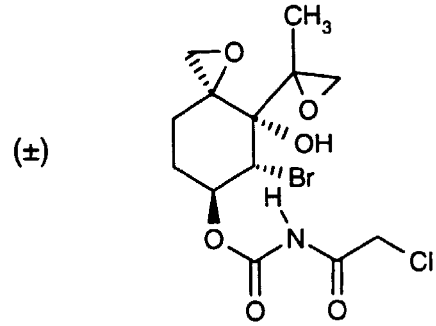

Diastéréoisomère obtenu lors de la synthèse du composé de l'exemple 4.

Analyse élémentaire :

| (Formule brute $C_{13}H_{17}BrClNO_6$ masse moléculaire : 398,64) | | | |
|---|---|---|---|
| | **C** | **H** | **N** |
| **% trouvé** | 39,02 | 4,23 | 3,69 |
| **% calculé** | 39,17 | 4,30 | 3,51 |

**EXEMPLE 39 : (3*S**, 4*S**, 6*R**)-4,6-Dichloroacétylcarbamoyloxy-4-(5-phénylpentyl)-1-oxa-*spiro*[2.5]octane**

(±) 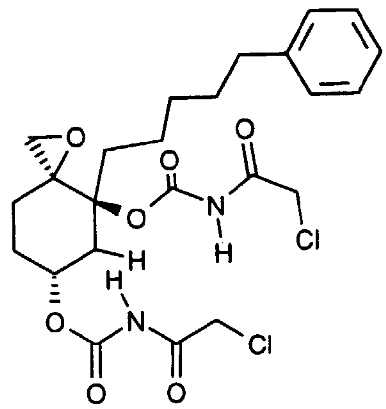

En procédant comme décrit pour l'exemple 12 à partir de l'isomère D obtenu à l'étape B de l'exemple 10, le composé titre est obtenu.

Analyse élémentaire :

| (Formule brute : $C_{24}H_{30}Cl_2N_2O_7$ masse moléculaire : 529,42) | | | |
|---|---|---|---|
| | **C** | **H** | **N** |
| **% trouvé** | 54,25 | 5,94 | 5,47 |
| **% calculé** | 54,45 | 5,71 | 5,29 |

**EXEMPLE 40 : (3*S**, 4*R**, 6*R**)-4,6-Di(2-chloropropanoyl)carbamoyloxy-4-(5-phénylpentyl)-1-oxa-*spiro*[2.5]octane**

(±) 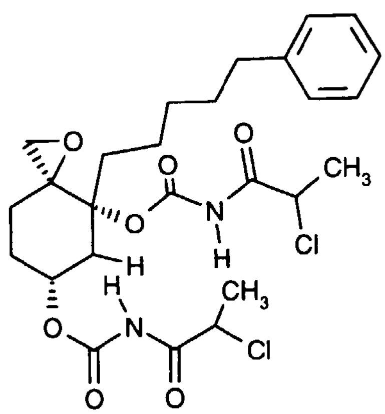

En procédant comme décrit pour l'exemple 12 à partir de l'isomère A obtenu à l'étape B de l'exemple 10, puis en remplaçant l'isocyanate de chloroacétyle par l'isocyanate de 2-chloropropanoyle, le produit titre est obtenu.

**EXEMPLE 41 : Benzyl-[(1*R**, 4*S**)-(1,4-dichlorocarbamoyloxy-2-(5-phénylpentyl) cyclohex-2-ényl)méthyl]méthylsulfonium**

(±)

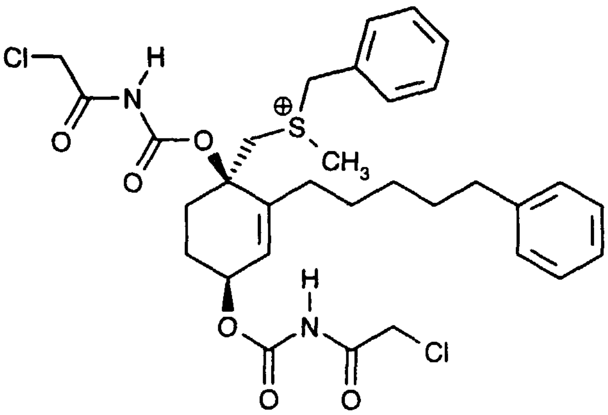

**EXEMPLE 42 : (3*S**, 4*S**)-4-[(1*E*)-hex-1-ényl]-4-hydroxy-1-oxa-*spiro*[2.5]octan-6-one**

(±)

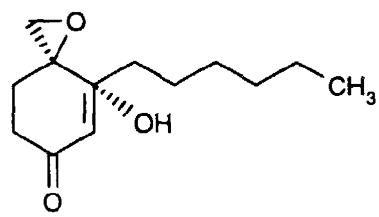

Etape A : 4-(hex-1-ynyl)-4-hydroxy-6-méthoxy-1-oxa-*spiro*[2.5]oct-5-ène

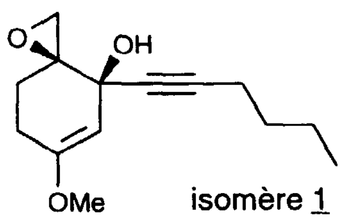

isomère 1

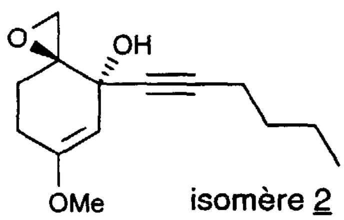

isomère 2

A une solution de 17,16 g (208 mmol) d'1-hexyne dans 800 ml de diéthyléther anhydre refroidie à -78°C sont ajoutés goutte à goutte et sous atmosphère d'azote 130 ml (207 mmol) de n-butyllithium (1.6 M dans l'hexane). L'ensemble est laissé sous agitation à -78°C pendant 15 minutes puis à 0°C pendant 1 heure. A cette solution est alors additionnée goutte-à-goutte une solution de 16 g de 6-méthoxy-1-oxa-*spiro*[2.5]oct-5-én-4-one (104 mmol) dans 100 ml de toluène anhydre refroidie à -78°C. Après 4 heures d'agitation à -78°C, le mélange réactionnel est versé dans une solution aqueuse à 10% de chlorure d'ammonium. Le traitement habituel de la phase organique fournit après chromatographie sur gel de silice (éluant : pentane/acétate d'éthyle, 4:1) 10,4 g (44,01 mmol) d'isomère 1 et 13 g (55,01 mmol) d'isomère 2.

Rendement : 95%

Etape B :4-(hex-1-ényl)-4-hydroxy-6-méthoxy-1-oxa-*spiro*[2.5]oct-5-ène

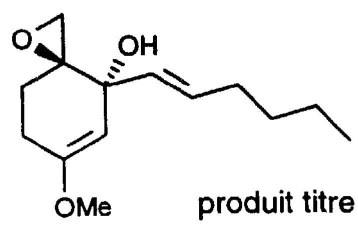
produit titre

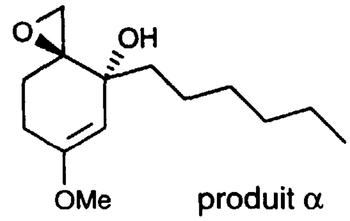
produit α

L'hydrogénation d'une solution de 13 g (55,01 mmol) d'isomère 2 obtenu à l'étape A dans 500 ml de benzène en présence de 7,5 g de catalyseur de Lindlar pendant 7 heures conduit après filtration et concentration à 13 g (54,09 mmol) d'un mélange du produit titre et du produit α que l'on utilise sans purification pour l'étape suivante.

Etape C : 5-bromo-6,6-diméthoxy-4-(hex-1-ényl)-4-hydroxy-1-oxa-*spiro*[2.5] octane

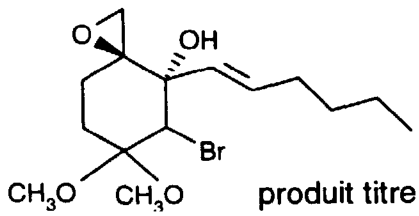
produit titre

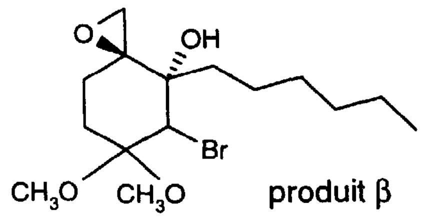
produit β

En procédant selon le mode opératoire décrit à l'étape B de l'exemple 1 à partir de 13 g (54,09 mmol) du mélange de produits obtenu à l'étape B et de 9,6 g (54,66 mmol) de N-bromosuccinimide dans 500 ml de méthanol, 17,3 g (49,25 mmol) d'un mélange de produit titre et de produit β est obtenu, mélange que l'on utilise sans purification pour l'étape suivante.

Etape D : 6,6-diméthoxy-4-(hex-1-ényl)-4-hydroxy-1-oxa-*spiro*[2.5]octane

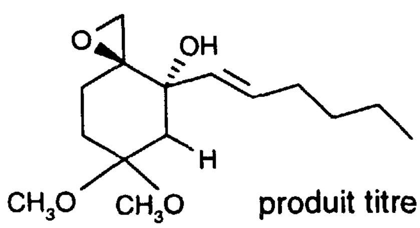
produit titre

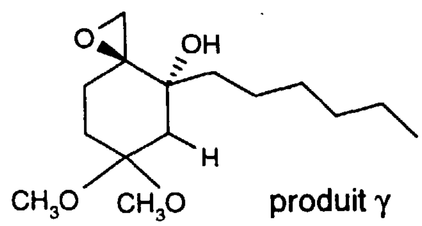
produit γ

En procédant selon le mode opératoire décrit à l'étape A de l'exemple 5 à partir de 17,3 g (49,25 mmol) du mélange de produits obtenu à l'étape C et de 23,6 ml (88,89 mmol) d'hydrure de tributylétain dans 23,6 ml de toluène anhydre, 13,4 g (49,56 mmol) d'un mélange de produit titre et de produit γ que l'on utilise sans purification pour l'étape suivante.

Etape E : (3*S**, 4*S**)-4-[(1*E*)-hex-1-ényl]-4-hydroxy-1-oxa-*spiro*[2.5]octan-6-one

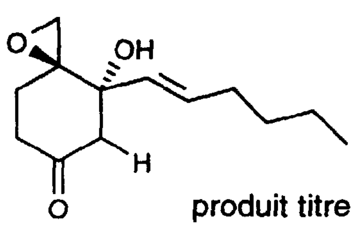
produit titre

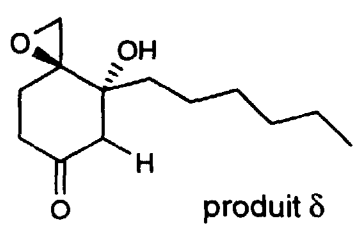
produit δ

En procédant selon le mode opératoire décrit à l'étape C de l'exemple 1, à partir de 13,4 g (49,56 mmol) du mélange

de produits obtenu à l'étape D et de 9,4 g (49,27 mmol) d'acide paratoluènesulfonique dans 500 ml d'un mélange acétone/eau (3:2) ; 1,19 g (5,3 mmol) de produit titre et 1,7 g (7,51 mmol) de produit δ sont obtenus après chromatographie sur phase inverse (HPLC) (éluant : acétonitrile/eau, 40:60).
Rendement : 11%

Analyse élémentaire :

| (Formule brute : $C_{13}H_{20}O_3$ masse moléculaire : 224,30) | | |
|---|---|---|
| | **C** | **H** |
| **% trouvé** | 68,93 | 8,96 |
| **% calculé** | 69,91 | 8,99 |

**EXEMPLE 43 : (3*S**, 4*R**)-4-hexyl-4-hydroxy-1-oxa-spiro[2.5]octan-6-one**

(±) 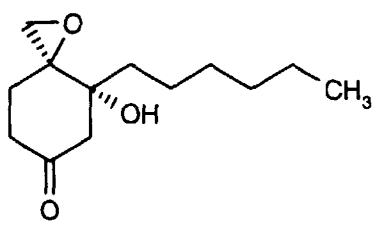

Produit δ obtenu lors de la synthèse décrite à l'exemple 42.

**ETUDE PHARMACOLOGIQUE**

**EXEMPLE A : Etude de l'activité anti-proliférative des composés de l'invention**

Trois lignées cellulaires ont été utilisées :

- 1 leucémie murine, L1210,
- 1 carcinome épidermoïde humain, A431,
- 1 culture primaire de cellules endothéliales d'aorte de porc, CEAP.

Les cellules sont cultivées dans du milieu de culture RPMI 1640 complet contenant 10% de sérum de veau foetal, 2 mM de glutamine, 50 unités/ml de pénicilline, 50 μ g/ml de streptomycine et 10 mM d'HEPES (pH = 7,4).

Les cellules sont réparties dans des microplaques et exposées aux composés cytotoxiques. Les cellules sont ensuite incubées pendant deux jours (L1210), 3 jours (CEAP) et 4 jours (A431). Le nombre de cellules viables est ensuite quantifié par un essai colorimétrique, le Microculture Tetrazolium Assay (Carmichael J., DeGraff W. G., Gazdar A. F., Minna J. D. and Mitchell J. R., Evaluation of a tetrazolium-based semiautomated colorimetric assay : assessment of chemosensitivity testing, *Cancer Res., 47*, 936-942, (1987)).

Les composés de la présente invention ont montré une activité anti-proliférative sur ces trois lignées cellulaires.

A titre d'exemple, les $IC_{50}$ (concentrations en composé qui inhibent à 50 % la prolifération des cellules traitées) sont selon les lignées cellulaires, de 3 à 10 fois inférieures à celles de la fumagilline.

**EXEMPLE B : Inhibition de la néovascularisation de la membrane chorioallantoidïenne d'embryon de poulet**

Ce test est réalisé avec des embryons de poulet comme décrit précédemment (Crum R., Szabo S. and Folkman J., *Science*, (1985), *230*, 1375-1378). Les oeufs fécondés (J0) sont incubés à 37°C. Une poche d'air est créée en prélevant 1 ml d'albumine (J3), puis une fenêtre est découpée dans la coquille (J4) et la membrane vitelline est enlevée pour dégager la membrane chorio-allantoïdienne (MCA).

Les produits à tester sont solubilisés dans de l'éthanol et déposés sur des disques de méthylcellulose qui sont séchés et déposés sur la MCA au jour 6. Entre 8 et 16 oeufs sont utilisés par groupe. La zone située autour du disque est ensuite examinée 48 heures plus tard. Les oeufs présentant une zone avasculaire supérieure à 4 mm de diamètre

sont dénombrés et les résultats sont exprimés en pourcentage d'oeufs présentant une zone avasculaire. Les résultats obtenus pour chacun des composés de l'invention sont indiqués dans le tableau 1 suivant :

**- Tableau 1 -**

**Inhibition de la néovascularisation de la membranne chorioallantoïdienne d'embryon de poulet**

| Composé | % d'oeufs présentant une zone avasculaire |
|---|---|
| Exemple 6 | 79 ± 9 |
| Exemple 7 | 81 ± 10 |
| Exemple 10 | 73 ± 11 |
| Exemple [illegible] | 70 ± 16 |
| Exemple 17 *Diastéréoisomère A* | 74 ± 7 |
| Exemple 18 | 69 ± 6 |
| Exemple 19 | 76 ± 9 |
| Exemple 21 | 76 ± 6 |
| Exemple 23 | 78 ± 7 |
| Exemple 28 | 90 ± 4 |
| Exemple 29 | 66 ± 6 |
| Exemple 30 *Diastéréoisomère A* *Diastéréoisomère B* | 75 ± 7<br>68 ± 5 |

**EXEMPLE C : Activité antitumorale**

L'activité antitumorale des composés de l'invention a été étudiée selon le protocole décrit par R.I. Geran et coll., *Cancer Chemotherapy Reports,* (1972), Part 3, pages 3 sqq.

Des souris ont été reparties au hasard en groupes traités (11 souris/groupe) et en un groupe contrôle de 40 souris.

Des fragments tumoraux ont été implantés au jour 0 (implant sous cutané). Les composés à tester ont été administrés pendant 12 jours (jour 1 à jour 12) par voie i.p.

Le poids moyen de la tumeur a été déterminé au jour 13 après implantation. Le pourcentage d'inhibition a été calculé selon la formule :

$$\% \text{ inhibition} = 100 - \frac{\text{poids moyen de la tumeur (groupe traité)}}{\text{poids moyen de la tumeur (groupe témoin)}}$$

A titre d'exemple le composé de l'exemple 27 a fourni les résultats suivants :

| Dose (mg/kg) | % inhibition |
|---|---|
| 30 | 56 |
| 60 | 82 |

**EXEMPLE D : Composition pharmaceutique : comprimés**

Formule de préparation pour 1000 comprimés dosés à 50 mg.

| | |
|---|---|
| Composé de l'exemple 12 | 50 g |
| Amidon de blé | 15 g |
| Amidon de maïs | 15 g |
| Lactose | 65 g |
| Stéarate de magnésium | 2 g |
| Silice | 1 g |
| Hydroxypropylcellulose | 2 g |

## Revendications

**1.** Composés de formule générale (I) :

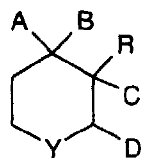

(I)

dans laquelle :

- R est choisi parmi les radicaux :

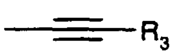 , 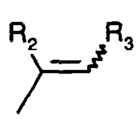 , 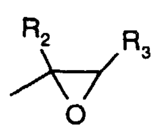 , et 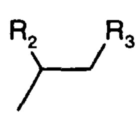

(α) (β) (γ) (δ)

- **A** et **B** sont tels que :
  **A** représente le radical méthyle, et **B** représente le radical $-OR_1$,
  ou bien,
  et seulement dans le cas où R représente le radical (δ), **A** représente l'hydrogène et **B** est choisi parmi

le radical $-CH_2-OH$ et le radical $-CH_2-OR_1$,
ou bien,
**A** représente le radical

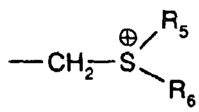

et **B** est choisi parmi le radical hydroxy, et le radical $-OR_1$,
ou bien,
**A** et **B** forment ensemble avec l'atome de carbone qui les porte et le groupement $-CH_2-O-$, un cycle oxirane,

- **C** et **D** sont tels que :
  **C** représente le radical hydroxy et **D** est choisi parmi l'hydrogène et le brome,
  ou bien,
  et seulement dans le cas où R représente le radical (δ), **C** représente le radical $OR_1$ et **D** est choisi parmi l'hydrogène et le brome,
  ou bien, et seulement dans le cas où R représente le radical (δ), **C** et **D** représentent chacun simultanément l'hydrogène, ou bien,
  **C** et **D** forment ensemble avec les atomes de carbone qui les portent et avec le groupement -O-, un cycle oxirane, ou bien forment ensemble avec les atomes de carbone qui les portent et le groupement -O-CO-O-, un hétérocycle dioxygéné, ou bien forment ensemble une liaison,

- **Y** est choisi parmi le radical -CO- et le radical $-CH(OR_1)-$,

- $\mathbf{R_1}$ représente le radical

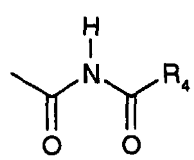

,

- $\mathbf{R_2}$ est choisi parmi l'hydrogène, un radical alkyle éventuellement substitué, et un radical aryle éventuellement susbtitué,

- $\mathbf{R_3}$ est choisi parmi l'hydrogène, un radical alkyle éventuellement substitué et un radical arylalkyle éventuellement substitué,

- $\mathbf{R_4}$ est choisi parmi un radical alkyle éventuellement substitué, le radical amino, un radical alkylamino éventuellement substitué, un radical dialkylamino éventuellement substitué, un radical aryle éventuellement substitué, un radical arylalkyle éventuellement substitué, un radical hétéroarylalkyle éventuellement substitué, et un radical alkényle éventuellement substitué,

- $\mathbf{R_5}$ représente un radical alkyle éventuellement substitué,

- $\mathbf{R_6}$ est choisi parmi un radical alkyle éventuellement substitué, un radical aryle éventuellement substitué, un radical arylalkyle éventuellement substitué, un radical hétéroaryle éventuellement substitué et un radical hétéroarylalkyle éventuellement substitué,

étant entendu que :

- la chaîne alkyle dans les termes « alkyle », « alkylamino », « dialkylamino », « arylalkyle » et « hétéroarylalkyle » désigne une chaîne hydrocarbonée saturée contenant de 1 à 6 atomes de carbone en chaîne droite ou ramifiée,

- le terme « aryle » désigne un radical choisi parmi phényle et naphtyle,

- le terme « hétéroaryle » désigne un radical choisi parmi pyridyle, quinolyle, isoquinolyle, imidazolyle, indolyle et isoindolyle,

- le terme « alkényle » désigne un radical choisi parmi vinyle et isopropényle,

- le terme « éventuellement substitué » associé aux radicaux alkyle, alkylamino, dialkylamino, aryle, arylalkyle, hétéroaryle, hétéroarylalkyle et alkényle, signifie que ces radicaux peuvent être éventuellement substitués, sur les parties acycliques et/ou, le cas échéant, sur les parties cycliques, par une ou plusieurs entités chimiques choisies parmi :

  - hydroxy,
  - halogène, choisi parmi fluor, chlore, brome et iode,
  - trihalogénométhyle,
  - nitro,
  - amino, alkylamino, dialkylamino
  - alkoxy, linéaire ou ramifié comportant de 1 à 6 atomes de carbone,
  - carboxy,
  - alkoxycarbonyle, linéaire ou ramifié comportant de 1 à 6 atomes de carbone, et,
  - acyle, linéaire ou ramifié comportant de 1 à 6 atomes de carbone,

  ainsi que leurs éventuels isomères géométriques leurs éventuels diastéréoisomères et leurs éventuels énantiomères sous forme pure ou sous forme de mélange.

**2.** Composés selon la revendication 1 pour lesquels Y représente le radical $-CH(OR_1)-$,
et leurs éventuels isomères géométriques, leurs éventuels diastéréoisomères et leurs éventuels énantiomères sous forme pure ou sous forme de mélange.

**3.** Composés selon la revendication 1 pour lesquels Y représente le radical -CO-, et leurs éventuels isomères géométriques, leurs éventuels diastéréoisomères et leurs éventuels énantiomères sous forme pure ou sous forme de mélange.

**4.** Composés selon la revendication 1 pour lesquels A et B forment ensemble un cycle oxirane, avec l'atome de carbone qui le porte et avec le groupement $-CH_2-O-$, ainsi que leurs éventuels isomères géométriques, leurs éventuels diastéréoisomères et leurs éventuels énantiomères sous forme pure ou sous forme de mélange.

**5.** Composé selon la revendication 1 qui est le (3*S*, 4*R*, 6*R*)-4,6-dichloroacétylcarbamoyloxy-4-(5-phénylpentyl)-1-oxa-*spiro*[2.5]octane,
ses diastéréoisomères (3*S*, 4*R*, 6*S*), (3*S*, 4*S*, 6*R*) et (3*S*, 4*S*, 6*S*) ainsi que leurs énantiomères sous forme pure ou sous forme de mélange.

**6.** Composé selon la revendication 1 qui est le (3*S*, 4*S*, 6*S*)-4,6-dichloroacétylcarbamoyloxy-4-hexyl-1-oxa-*spiro*[2.5] octane,
ses diastéréoisomères (3*S*, 4*S*, 6*R*), (3*S*, 4*R*, 6*R*) et (3*S*, 4*R*, 6*R*) ainsi que leurs énantiomères sous forme pure ou sous forme de mélange.

**7.** Composé selon la revendication 1 qui est le (3*S*, 4*S*, 6*R*)-4,6-dichloroacétylcarbamoyloxy-4-isopropyl-1-oxa-*spiro* [2.5]octane
ses diastéréoisomères (3*S*, 4*S*, 6*S*), (3*S*, 4*R*, 6*R*) et (3*S*, 4*R*, 6*S*) ainsi que leurs énantiomères sous forme pure ou sous forme de mélange.

**8.** Composé selon la revendication 1 qui est le 5-chloroacétylcarbamoyloxy-2-(2-*spiro*-oxiranyl)-1-(4-méthylpentyl)-7-oxabicyclo[4.1.0]heptane,
ses diastéréoisomères ainsi que leurs énantiomères sous forme pure ou sous forme de mélange.

**9.** Composé selon la revendication 1 qui est le 5-bromo-6-chloro-acétylcarbamoyloxy-4-hydroxy-4-(5-phénylpentyl)-1-oxa-*spiro*[2.5]octane,

ses diastéréoisomères ainsi que leurs énantiomères sous forme pure ou sous forme de mélange.

**10.** Composé selon la revendication 1 qui est le (3*S*, 4*R*)-4-chloroacétylcarbamoyloxy-4-(5-phénylpentyl)-1-oxa-*spiro* [2.5]octan-6-one, son diastéréoisomère (3*S*, 4*S*) ainsi que leurs énantiomères sous forme pure ou en mlélange.

**11.** Procédé de préparation des composés de formule (I), caractérisé en ce que le composé de formule (II):

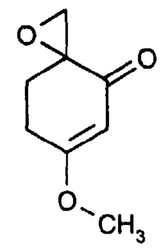

(II)

est soumis, selon le composé de formule (I) souhaité, à la combinaison d'une ou plusieurs des réactions suivantes :

***a) Pour l'insertion du radical R tel que défini dans la formule (I),***
par addition, en présence de butyl-lithium, en solvant polaire anhydre, sous atmosphère inerte, à température comprise entre -78°C et 0°C, d'un composé de formule (III) ou d'un composé de formule (IV) :

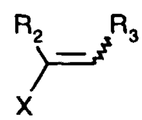

$HC\equiv C-R_3$

(III) (IV)

dans lesquelles $R_2$ et $R_3$ sont tels que définis dans la formule (I) et X représente un atome d'halogène afin d'obtenir respectivement les composés pour lesquels R représente respectivement (β) ou (α), les composés pour lesquels R représente (α) ou (β) définis précédemment pouvant être soumis à hydrogénation catalytique, pour conduire aux composés pour lesquels R représente (δ) tel que défini dans la formule (I), les composés pour lesquels R représente (α) défini dans la formule (I) pouvant être soumis à l'action d'un agent oxydant, de façon à obtenir les composés pour lesquels R représente (y) défini dans la formule (I),

***b) Pour le radical Y défini dans la formule (I)***
par transformation de la fonction méthoxy du composé de formule (II) en fonction cétone lors de l'insertion du radical R décrite ci-dessus,
les composés pour lesquels Y représente -CO- pouvant, si on le souhaite, être soumis à l'action d'un réducteur, pour obtenir l'alcool correspondant qui est soumis à l'action d'un isocyanate de formule (V) :

$$R_4\text{-CONCO (V)}$$

dans laquelle $R_4$ est tel que défini dans la formule (I),
de manière à obtenir les composés pour lesquels Y représente $-CH(OR_1)-$, $R_1$ étant tel que défini dans la formule (I),

***c) Pour les différentes valeurs de A et B,***

- pour les composés de formule (I) pour lesquels A et B forment ensemble avec l'atome de carbone qui les porte et le groupement $-CH_2-O-$, un cycle oxirane, sans aucune transformation, ce cycle oxirane étant déjà présent dans le composé de départ de formule (II) défini ci-dessus,

- pour les composés de formule (I) pour lesquels A représente le radical méthyle et B représente le radical $-OR_1$, par réaction du cycle oxirane présent dans le composé de formule (II) précédement défini, selon la réaction suivante :

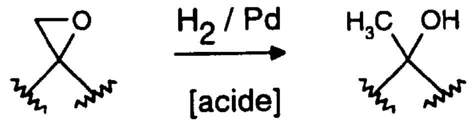


- pour les composés de formule (I) pour lesquels R représente le radical (δ) défini précédemment, A représente l'hydrogène et B est choisi parmi le radical $-CH_2-OH$ et le radical $-CH_2-OR_1$, par réaction du cycle oxirane présent dans la formule (II) précédemment définie, selon les réactions suivantes :

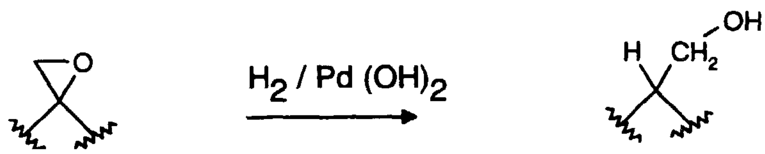


(VI)

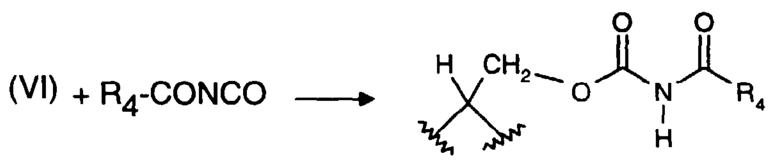


où $R_4$ est tel que défini dans la formule (I),

- pour les composés de formule (I) pour lesquels A représente le radical

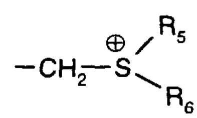

et B est choisi parmi le radical hydroxy et le radical $-OR_1$, par réaction du cycle oxirane présent dans la formule (II) précédemment définie selon les réactions suivantes :

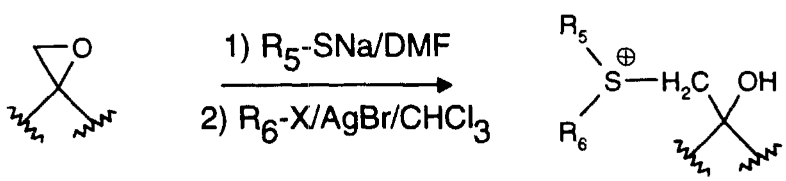


(VII)

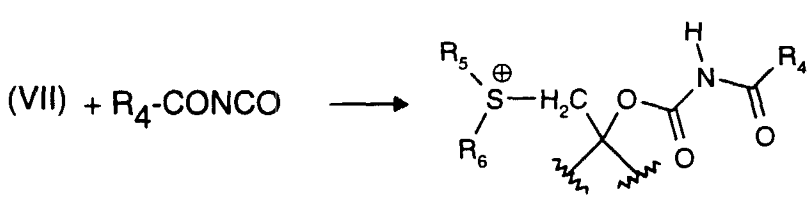


où $R_4$, $R_5$ et $R_6$ sont tels que définis dans la formule (I), et X représente un atome d'halogène,

***d) Pour les différentes valeurs de C et D,***

- pour les composés de formule (I) pour lesquels C représente le radical hydroxy et D représente le brome, par réaction de l'intermédiaire de synthèse de formule (VIII) obtenu lors de l'insertion du radical R (voir paragraphe a) ci-dessus) avec un agent de bromation, selon la réaction suivante :

(VIII) 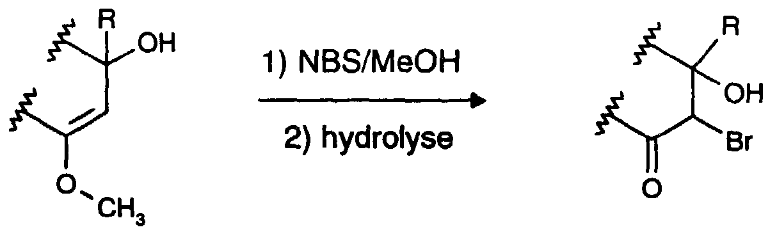

(IX)

où R est tel que défini précédemment,
- pour les composés de formule (I) pour lesquels C représente le radical hydroxy et D représente l'hydrogène, par réaction de l'intermédiaire de synthèse de formule (VIII) défini précédemment, selon la réaction suivante :

(VIII) 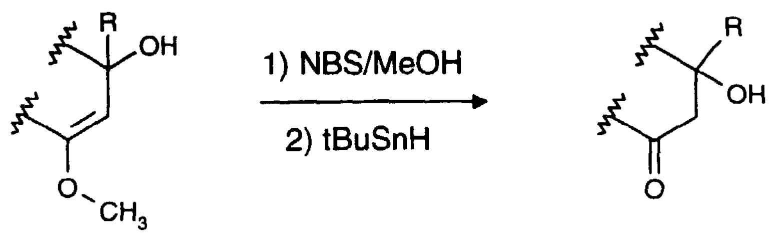

(X)

où R est tel que défini précédemment,
les fonctions hydroxy des composés de formules (IX) et (X) définis ci-dessus pouvant être carbamoylées sous l'action de l'isocyanate de formule $R_4$-CONCO, $R_4$ étant tel que défini précédemment, de façon à obtenir les composés de formule (I) pour lesquels D est choisi parmi l'hydrogène et le brome et C représente le radical $OR_1$
- les composés de formule (I) pour lesquels les valeurs de C et D étant autres que celles définies ci-dessus étant obtenus à partir du composé de formule (VIII) selon le schéma suivant :

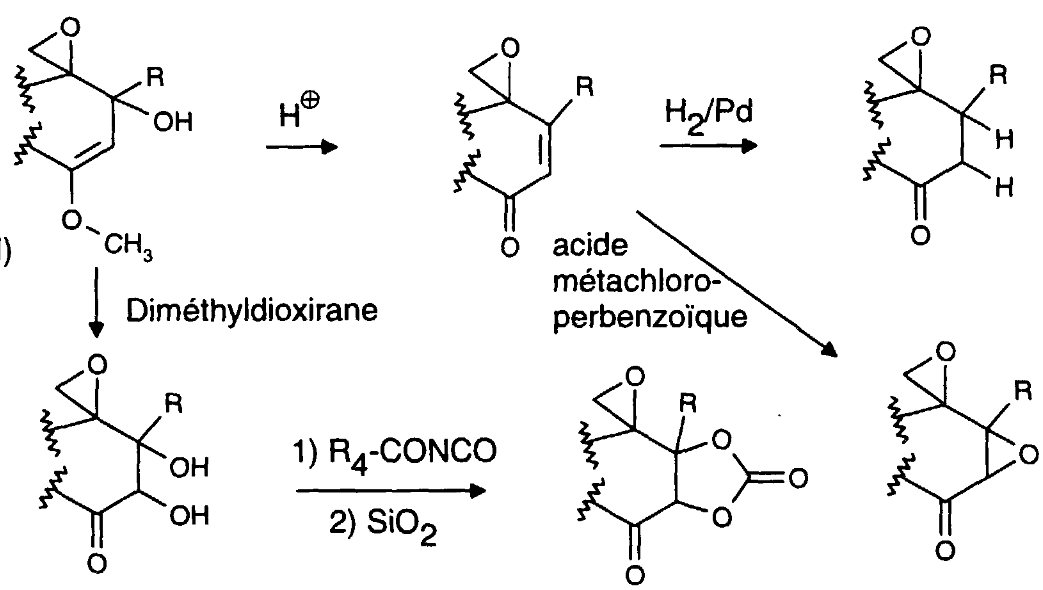

(VIII)

où R et $R_4$ sont tels que définis précédemment,

l'ensemble des intermédiaires de synthèse ainsi que les composés de formule (I) pouvant éventuellement être purifiés par une technique classique de purification et séparés si on le désire et le cas échéant en isomères géométriques et/ou diastéréoisomères et/ou énantiomères par une technique classique de séparation.

**12.** Compositions pharmaceutiques contenant comme principe actif au moins un composé selon l'une quelconque des revendications 1 à 10, seul ou en combinaison avec un ou plusieurs véhicules inertes, non toxiques et pharmaceutiquement acceptables.

**13.** Compositions pharmaceutiques selon la revendication 12 exerçant une activité inhibitrice de l'angiogenèse, et utiles dans le traitement des affections dues ou reliées aux troubles de l'angiogenèse.

Office européen des brevets

# RAPPORT DE RECHERCHE EUROPEENNE

Numero de la demande
EP 96 40 0872

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A,D | EP-A-0 357 061 (TAKEDA CHEMICAL INDUSTRIES LTD ;CHILDRENS MEDICAL CENTER (US)) 7 Mars 1990<br>* revendications; exemples *<br>--- | 1-10 | C07D303/16<br>A61K31/335<br>C07D411/08<br>C07C271/64<br>//(C07D411/08, 303:16,317:64) |
| A | EP-A-0 354 787 (FUJISAWA PHARMACEUTICAL CO) 14 Février 1990<br>* revendications; exemples *<br>--- | 1-10 | |
| A | EP-A-0 354 767 (FUJISAWA PHARMACEUTICAL CO) 14 Février 1990<br>* revendications; exemples *<br>--- | 1-10 | |
| D,A | PATENT ABSTRACTS OF JAPAN<br>vol. 013, no. 562 (C-665), 13 Décembre 1989<br>& JP-A-01 233275 (TAISHO PHARMACEUT CO LTD), 19 Septembre 1989,<br>* abrégé *<br>----- | 1-10 | |

| DOMAINES TECHNIQUES RECHERCHES (Int.Cl.6) |
|---|
| C07D<br>A61K<br>C07C |

Le présent rapport a été établi pour toutes les revendications

| Lieu de la recherche | Date d'achèvement de la recherche | Examinateur |
|---|---|---|
| LA HAYE | 26 Juin 1996 | Pauwels, G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)